(19) Europäisches Patentamt
European Patent Office
Office européen des brevets

(11) Publication number: **0 141 269**
**B1**

(12) # EUROPEAN PATENT SPECIFICATION

(45) Date of publication of patent specification: **23.08.89**

(51) Int. Cl.⁴: **C 08 F 8/44,** C 08 F 8/32

(21) Application number: **84111729.4**

(22) Date of filing: **01.10.84**

(54) **Modified polyvinyl alcohol polymers.**

(30) Priority: **07.10.83 US 540041**
**07.10.83 US 540145**

(43) Date of publication of application:
**15.05.85 Bulletin 85/20**

(45) Publication of the grant of the patent:
**23.08.89 Bulletin 89/34**

(84) Designated Contracting States:
**AT BE CH DE FR GB IT LI LU NL SE**

(56) References cited:
**DE-A-2 050 785**
**US-A-3 345 346**
**US-A-3 348 997**

**CHEMICAL ABSTRACTS, vol. 98, no. 24, June 1983, Columbus, OH (US); p. 29, no. 199187t**

(73) Proprietor: **ICI AMERICAS INC.**
**Concord Pike & New Murphy Road**
**Wilmington Delaware 19897 (US)**

(72) Inventor: **Davis, Ronald Ivey**
**22 Crestfield Road**
**Wilmington, DE (US)**
Inventor: **Phalangas, Charalambos John**
**8 Chilton Road**
**Edenridge, DE (US)**
Inventor: **Titus, George Robert**
**815 Chaplain Avenue**
**Wilmington, DE (US)**
Inventor: **Restaino, Alfred Joseph**
**615 Black Gates Road**
**Wilmington, DE (US)**

(74) Representative: **Baillie, Iain Cameron et al**
**c/o Ladas & Parry Isartorplatz 5**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention is directed to skin conditioning polymers which when applied to skin form retentive films which aid in reducing moisture loss. The invention relates in general to film forming polyvinyl alcohol polymer derivatives and specifically to those having certain amine and quaternary ammonium nitrogen containing pendant groups or a combination of these nitrogen containing groups. Of particular interest are polymers having a polyvinyl alcohol backbone or base chain with randomly distributed pendant substituent nitrogen containing groups comprising at least two oxygen linked groups selected from tertiary amines, quaternary ammonium, alkyl, arylalkyl, hydroxy alkyl, alkyl acids and hydroxy alkyl acids.

Unites States Patent No. 3,345,346 discloses the reaction product of polyvinyl alcohol with an alkoxy methylamine to add nitrogen-containing oxygen-linked pendant chains to the polyvinyl alcohol chain. Such products may be quaternized by reaction with, for example, alkyl p-toluene sulfonates or alkyl chloride. The products are taught to be useful as mordants, silver halide peptizers, sensitizers and antistatic agents.

As a result of the presence of quaternary ammonium groups in the polymer, thin film coatings on animal skin penetrate the outer layers of the skin to provide sufficient adhesive properties while remaining sufficiently elastomeric to avoid discomfort after drying. While the thin films act as a partially impenetrable barrier to prevent loss of moisture by evaporation they also behave as moisture retainers through the possible formation of hydrates at the quaternary ammonium sites and by inclusion of water molecules through hydrogen bonding on the hydrophilic polymer matrix. Other pendant groups on the polyvinyl alcohol chain selected from the tertiary amines and the alkyl groups further enhanced the comfort of the dry thin film on the skin while they provide means for adjusting the hydrophilic/lipophilic balance to provide for compatibility with specific solvents thereby permitting the polymer to be formulated in a wide number of skin conditioning, cosmetic and pharmaceutical formulations. Furthermore, the polymers act as suspending agents for insoluble pigments and pharmaceutical actives contained in such formulations in high concentrations.

The present invention provides a quaternary nitrogen or tertiary amine containing polyvinyl alcohol polymer base chain (having a number average molecular weight of at least 2,000 and preferably up to about 200,000 and higher when unmodified) and a multiplicity of oxy-linked pendant groups comprising $R_4$ groups and at least one group being selected from:

(a) $—R—N^+R_1R_2R_3A^-$,
(b) $—R—NR_1R_2$, groups

wherein

R is alkylene, substituted alkylene preferably hydroxyalkylene, or acylene of formula weight ranging from 14 to about 3,000,

$R_1$, $R_2$, $R_3$ are alkyl or arylalkyl radicals, having 1—20 carbon atoms which may be the same or different,

$A^-$ is an anion, and

$—R_4$ is alkyl, arylalkyl or specific substituents bearing alkyl or arylalkyl radicals such that the total nitrogen content in the resin polymer ranges from 0.01—7.0% by weight. Often type (a) groups will be present in combination with type (b) groups. Another object is to provide for a preferred process for the synthesis of such compositions. It is still another object to provide for aqueous solutions containing 0.1—30% by weight of polymers which are useful in skin and hair conditioning lotions, ointments, cosmetic and pharmaceutical formulations for application to hair, skin and nails and comprise modified polyvinyl alcohol polymer comprising oxygen-linked pendant groups selected from a) $—RN^+R_1R_2R_3A^-$ and b) a mixture of $—R—NR_1R_2$ and $R_4$ groups. Another object provides for a film forming polymer which also performs as a dispersant for insoluble particulates in such formulations.

R in the above formula may be selected from alkyl groups such as methylene, ethylene, propylene, butylene, pentylene, hexylene, ethylhexylene, dodecylene, tetradecylene, hexadecylene, octadecylene, and substituted alkyl groups such as hydroxypropylene, hydroxybutylene, acetyl, propionyl, butyryl, octadecanoyl, and octadecenoyl.

$R_1$, $R_2$, and $R_3$ may be selected from the alkyl groups such as methyl, ethyl, propyl, butyl, pentyl, hexyl, octyl, decyl, dodecyl, tridecyl, tetradecyl, hexadecyl, octadecyl, octadecenyl, phenyl or benzyl.

$A^-$ may be selected from a large number of anion such as chloride, bromide, iodide, hydroxide, lower alkyl (1—6 carbon atoms) sulfate, tetrafluoroborate, nitrate and perchlorate.

$R_4$ may be selected from a number of groups such as alkyl, alkylaryl, substituted alkyl and substituted alkylaryl radicals primarily alkyl or arylalkyl radicals bearing hydroxy or carboxyl groups or a combination of hydroxyl and carboxyl groups having a formula weight up to about 3000 and preferably less than 1000. The cumulative formula weight of the pendant groups are controlled in the synthesis of the polymer such that total nitrogen content ranges from 0.01—7% by weight. The overall proportion of $R_4$ groups added to the polyvinyl alcohol base chain are controlled such that their cumulative formula weight comprises from 0.5—50% by weight of the total product polymer weight and the ratio (b/a) of the cumulative formula weight of tertiary amine groups to the total formula weight of quaternary ammonium containing groups may vary from 0.90% by weight. Depending on the amount of the $—R—N^+R_1R_2R_3A^-$ and $—R_4$ groups the presence of $—R—N—R_1R_2$ groups may not be needed for certain applications. If the hydroxy or carboxy

bearing —$R_4$ type pendant groups are attached to the polyvinyl alcohol base chain prior to the reaction with quaternary ammonium or tertiary amine bearing groups some of the quaternary ammonium or tertiary amine groups may become oxy-linked to the —$R_4$ pendant group in place of being oxy-linked directly to the polyvinyl alcohol base chain.

Specific $R_4$ groups may be selected from methyl, ethyl, propyl, butyl, pentyl, hexyl, octyl, nonyldecyl, dodecyl, tridecyl, tetradecyl, hexadecyl, octadecyl, hydroxyethyl, mono or dihydroxypropyl, mono or polyhydroxybutyl, mono or polyhydroxypentyl, mono or polyhydroxyhexyl, mono or polyhydroxyoctyl, mono or polyhydroxypentyl, mono or polyhydroxyhexyl, mono or polyhydroxyoctyl, mono or polyhydroxydecyl, mono or polyhydroxydodecyl, mono or polyhydroxytetradecyl, mono or polyhydroxyhexadecyl, mono or polyhydroxyoxtadecyl, carboxymethylene, carboxyethylene, carboxypropylene, carboxybutylene, carboxypentylene, carboxyhexylene, carboxyheptylene, carboxyoctylene, carboxydecylene, carboxydodecylene, carboxytridecylene, carboxytetradecylene, carboxypentadecylene, carboxyhexadecylene, carboxyheptadecylene, carboxyoxtadecylene, carboxy(hydroxyethylene), carboxy(hydroxypropylene), carboxy(hydroxybutylene), carboxy(hydroxypentylene), carboxy(hydroxyhexylene), carboxy(hydroxyheptylene), carboxy(hydroxyoctylene), carboxy(hydroxydecylene), carboxy(hydroxydodecylene), carboxy(hydroxytridecylene), carboxy(hydroxytetradecylene), carboxy(hydroxypentadecylene), carboxy(hydrohexadecylene), carboxy(hydroxyheptadecylene), and carboxy(hydroxyoctadecylene).

The quaternary nitrogen containing polyvinyl alcohol polymer compositions of the invention may be represented by a typical polymer segment having the following idealized structural formula:

Type (a) groups include:

$$-[(-CH_2CHORN^+R_1R_2R_3A^-)(-CH_2CHOH-)_{n1}(CH_2CHORN^+R_1R_2R_3A^-)]_{n2}-$$

where

$n^2$=200—3000, and

$n^1$=0—600, and

R, $R_1$, $R_2$, $R_3$, and $A^-$ are the same as described above.

Illustrative of the types of numerous pendant quaternary groups linked by oxygen as randomly distributed units in the polyvinyl alcohol base chain may be given as follows:

(1) $-CH_2CH(OH)CH_2N^+(CH_3)_3Cl^-$

(2) $-(CH_2CH[CH_2N^+(CH_3)_3Cl^-]O)_nCH_2CH(OH)CH_2N^+(CH_3)_3Cl^-$
n=1—20

(3) $-(C{=}O)CH_2N^+(CH_2CH_3)_3Br^-$

(4) $-CH_2CH(OH)CH_2N^+(CH_3)_2(CH_2Ph)Cl^-$

(5) $-CH_2CH_2N^+(CH_3)(CH_2CH_3)(CH_2Ph)CH_3SO_4^-$
where Ph=phenyl.

In the case where a mixture of groups is present:

$$-[(-CH_2CHORN^+R_1R_2R_3A^-)(-CH_2CHOH)_{m1}(CH_2CHOR_4)(CH_2CHOH)_{m2}(CH_2CHORN^+R_1R_2R_3A^-)]- \text{ or}$$

$$-[(CH_2CHORN^+R_1R_2R_3A^-)(CH_2CHOH)_{p1}(CH_2CHORNR_1R_2)(CH_2CHOH)_{p2}(CH_2CHOR_4)(CH_2CHOH)_{p3}(CH_2CHORNR_1R_2R_3A^-)]-$$

$m_1$, $m_2$=0—650

$m_1+m_2$=≤650

$p_1$, $p_2$, $p_3$=0—650

$p_1+p_2+p_3$=≤650

R, $R_1$, $R_2$, $R_3$, $R_4$ and $A^-$ are the same as above.

Illustrative examples of the numerous quaternary and tertiary amine groups and non-nitrogen containing groups in the randomly distributed units in the polyvinyl alcohol base chain may be given as follows:

(1) $-CH_2CH(OH)CH_2N^+(CH_3)_3Cl^-$

(2) $-(CH_2CH[CH_2N^+(CH_3)_3Cl^-]O)_eCH_2CH(OH)CH_2N^+(CH_3)_3Cl^-$

where e=1—20

(3) $-(C{=}O)CH_2N^+(CH_2CH_3)_3Br^-$

(4) $—CH_2CH(OH)CH_2N^+(CH_3)_2(CH_2Ph)Cl^-$

(5) $—CH_2CH_2N^+(CH_3)(CH_2CH_3)(CH_2Ph)CH_3SO_4^-$

(6) $—CH[(CH_2)_7CH_3]CH(OH)(CH_2)_7CH_2OH$

(7) $—CH[(CH_2)_7CH_3]CH(OH)(CH_2)_7CH_2OCH_2CH(OH)CH_2N^+(CH_3)_3Cl^-$

(8) $—CH_2CH[CH_2N^+(CH_3)_3Cl^-]OCH[(CH_2)_7CH_3]CH(OH)(CH_2)_7CH_2OH$

(9) $—CH_2CH(OH)CH_3$

(10) $—CH_2CH(CH_3)[OCH_2CH(OH)CH_2N^+(CH_3)_3Cl^-$

(11) $—CH_2CHCH_2N^+(CH_3)_3Cl^-[OCH_2CH(OH)CH_3]$

(12) $—CH_2CH(OH)CH_2N(CH_2Ph)CH_3$

(13) $—CH_2CH[OCH_2CH(OH)CH_2N^+(CH_2Ph)CH_3]CH_2N^+(CH_3)_3Cl^-$

(14) $—CH_2CH[OCH_2CH(OH)CH_2N^+(CH_3)_3Cl^-]CH_2N(CH_2Ph)CH_3$

(15) $—CH[(CH_2)_7CH_3]CH[OCH_2CH(OH)CH_2N^+(CH_3)_3Cl^-](CH_2)_7CH_2O—CH_2CH(OH)CH_2N^+(CH_3)_3Cl^-$

(16) $—(C{=}O)(CH_2)_{16}CH_3$

(17) $—(C{=}O)(CH_2)_8CH(CH_2)_7CH_3$

(18) $—(C{=}O)(CH_2)_7CH{=}CH(CH_2)_7CH_3$

(19) $—(C{=}O)(CH_2)_7CH(OH)CH(OH)(CH_2)_7CH_3$

where Ph=phenyl radical.

Particularly useful compositions are those defined in claims 8—16 of the appended claims.

While the above structures serve to illustrate the types of pendant groups which can be added to the polyvinyl alcohol (PVA) base chain it is apparent to one skilled in the art that many other arrangements of similar chemical structure can be added. It has been found that each of the above types of groups used in combination with one or more of the others as a substituent on the polyvinyl alcohol base chain yields the desired combination of products having the right moisture barrier properties especially when the nitrogen content of the final product ranges from 0.01—7% by weight. Depending on the type of radical attached to the nitrogen of the quaternary groups the effective range of the nitrogen content could be even more specific. It has been found for example that when the $R_1$, $R_2$, $R_3$, in the general formulas are all methyl radicals the effective nitrogen content may range from 0.1—5% by weight. In the case where all pendant groups are of type (a) the nitrogen content of the final product is preferably 0.01 to 3% by weight, and in this case when all of $R_1$, $R_2$ and $R_3$ are methyl the effective nitrogen content is for 0.1 to 3% by weight.

While many techniques have been employed in the art to add substituent groups to vary the polymer charge or its hydrophilic-lipophilic balance our preferred methods for attaching the substituent groups to polyvinyl alcohol involve the reaction of hydroxyl groups of the polyvinyl alcohol base chain with an epoxy (oxirane) group, a halo-hydrin group in aqueous solution, or a lower molecular weight alkyl alcohol ester of the substituent in a dipolar aprotic solution or an acid halide in either a dipolar aprotic solvent or a two phase system in the presence of an appropriate acid or base catalyst.

While a number of methods may be utilized for the preparation of the PVA derivatives described in this invention, the use of non-aqueous solvents such as dimethyl formamide or similar polar materials is possible, but generally these solvents must be thoroughly removed from the final product. The use of aqueous solvents or mixed aqueous systems is preferable but, in this case, yields must be optimized because of competitive reactions of the quaternary ammonium compounds with water as well as PVA hydroxyls in the presence of catalyst. Improved yields can be obtained by increasing the PVA concentration in water, adding the oxirane compound as a concentrate and reducing to a minimum the amount of base used to catalyze the addition reaction. Salt formed during the reaction is preferentially removed from the final product, since it may have a deleterious effect on the skin moisture barrier properties and its formulation. It has also been found that the pH of the derivatized PVA may affect the skin moisture barrier properties as well as the substantivity to skin. It is generally desirable to work in a pH range between 2—10 preferably from 5—9.

Particularly suitable means for preparing compositions are specified in claims 18—31.

The product can be obtained in a dry form by precipitation, filtering, drying and grinding. The

precipitation is accomplished by adding the reaction mixture to a nonsolvent such as acetone, methanol, ethanol and the like. The product also finds use in the form of the aqueous solution or suspension which can preferably be obtained by dialyzing the reaction mass to free it from salts and low molecular weight unreacted intermediates. To obtain a better understanding of the preparative techniques found to be most satisfactory attention is drawn to the following generalized and specific preparations which are intended to illustrate but not limit the invention and wherein all proportions mentioned are based on weight unless otherwise specified.

General preparation I (for production of composition wherein pendant groups are of type (a))

A flask equipped with a water cooled condenser, mechanical stirrer and thermometer is charged with polyvinyl alcohol and distilled water. The polyvinyl alcohol (PVA) which is generally a commercially available product prepared by hydrolysis of polyvinyl acetate may have from 0 to 25% residual acetate groups, preferably from 2 to 15% and the number average molecular weight may range from 2,000 to 200,000 and higher and preferably from 25,000 to 150,000. In addition, and for the purpose of this invention, a polyvinyl alcohol base chain may include up to 25% by weight of another comonomer such as vinyl pyrrolidone, acrylic and methacrylic acids and esters thereof. The aqueous slurry is heated to 80—90°C. and held for 1 hour or until the polyvinyl alcohol is completely dispersed or solvated. A catalytic amount of aqueous base such as alkali hydroxide such as sodium or potassium hydroxide is then added and the solution cooled to 40—90°C. At this point 2,3-epoxypropyltrialkylammonium halide, either in aqueous solution or crystalline form, may be added incrementally or all at once. This reagent is typically used in 0.003—1.0 mol ratio preferably from 0.10—0.3 mol per mol of hydroxyl group on the polyvinyl alcohol base chain. The entire solution is then stirred for an additional period typically 4 hours at 60°C. after which the solid product may be recovered.

Recovery is accomplished by one of two general techniques. The solution can be poured into a polymer non-solvent to precipitate solid product from aqueous solution. Alternatively the solution may be dialyzed through a semiporous membrane and the purified aqueous polymer used as obtained or precipitated out.

The precipitation is preferably accomplished either in acetone or in methanol affording yields up to 90% or better by weight after drying. Depending on reaction conditions the precipitated products have a nitrogen content in the range of 0.01—7%.

In the purification by dialysis the reaction mixture is freed from all species below a certain molecular weight. Two methods may be used one a static and one dynamic. In the static method the reaction mixture is placed inside a commercial semi-porous dialysis tube and the tubes are submerged in distilled water typically for periods of 12 to 48 hours. The contents of the tubes are then recovered and the product may be used as is. In the dynamic system a pressure pump is used to move water from the reaction mixture through a semi-porous membrane. The water carried out any inorganics and low molecular weight organics. The resulting concentrated product stream is then collected.

General Preparation II (for production of composition wherein pendant groups are of type (a))

A flask equipped as described in General Preparation I is charged with polyvinyl alcohol and distilled water. The aqueous slurry is heated to 80—90°C. and held for one hour or until the polymer is completely solvated. A catalytic amount of aqueous alkaline hydroxide such as sodium or potassium hydroxide is added and the pot cooled to 60—65°C. At this point, (chlorohydroxypropyl)trimethylammonium halide, either in aqueous solution or crystalline form, is added either incrementally or all at once. This reagent is typically used in 0.003—1.0 mole ratio preferably from 0.10—0.5 mole per mole of hydroxyl group on the polyvinyl alcohol base chain. The entire solution is then stirred at 40—90°C. for an additional period, typically four hours. The product is then recovered by one of the methods described in General Preparation I.

General Preparation III (for production of composition wherein pendant groups are of type (a))

In preparing quaternary ammonium groups linked to the polyvinyl alcohol base chain through ester linkages it is preferred to prepare them using a transesterification technique. In a typical reaction a four-neck one liter round bottom flask equipped with a thermometer, mechanical stirrer, water cooled condenser and a nitrogen sparger is charged with polyvinyl alcohol as described in the above General Preparation I, dimethylformamide, a trialkylalkoxycarbonyl ammonium halide, and a small amount of a transition metal salt as catalyst as manganese acetate for example. The entire dispersion is then heated to a temperature of 100—110°C. for 2 to 24 hours preferably 3 to 6 hours. Nitrogen is used as a carrier gas to remove the alcohol formed during the esterification reaction. The solution is then cooled to ambient temperature and poured into a polymer nonsolvent such as for example methanol, ethanol or acetone. The resulting precipitate is collected by filtration, washed, shredded mechanically and dried under vacuum to afford a nearly white product with a desired nitrogen content.

General Preparation IV (for production of composition wherein pendant groups of type (a))

A flask equipped as described in General Preparation I is charged with polyvinyl alcohol and distilled water. The aqueous slurry is heated to 80—90°C. and held for one hour or until the polymer is completely

dispersed or solvated. A catalytic amount of an acid such as sulfuric acid or aluminum hydrosilicate or any proton a acid or Lewis acid is added and the pot cooled to 40—90°C. At this point 2,3-epoxypropyltrialkylammonium halide may be added incrementally or all at once. This reagent is typically used in 0.003—1.0 mole ratio preferably from 0.1—0.3 mol per mol of hydroxyl group on the polyvinyl alcohol base chain. The entire solution is then stirred for an additional period typically 4 hours at 60°C. after which the solid product may be recovered by one of the methods described in General Preparation I.

General Preparation V (to produce composition with a mixture of pendant groups)

A flask equipped with a water cooled condenser mechanical stirrer and thermometer is charged with polyvinyl alcohol and distilled water to form an aqueous suspension or slurry. The polyvinyl alcohol which is generally a commercially available product prepared by hydrolysis of polyvinyl acetate may have from 0—35% by weight residual acetate groups and preferably from 2—15% by weight. The number average molecular weight of the starting material may range from 2,000 up to 200,000 and higher and preferably from 25,000—150,000. In addition and for the purpose of this invention, a polyvinyl alcohol base chain may include up to 25% by weight of another comonomer such as vinylpyrrolidone, acrylic and methacrylic acid and esters thereof.

The aqueous slurry is heated to 80—100°C. and then a catalytic amount of an alkaline hydroxide such as sodium or potassium hydroxide or acidic material when appropriate as a catalyst is added. The solution is then cooled to near ambient temperature if the addition of propylene oxide is intended or 50—75°C. when fatty epoxides or tertiary amine epoxides are to be added. These reagents may be used either singly or in combination in amounts ranging from 0—0.95 mol per mol of hydroxyl groups on the polyvinyl alcohol chain and preferably from 0.001—0.5 mol per mol of hydroxyl on the base chain. The entire solution is then generally treated with a quaternary ammonium reagent when used at a temperature of 80—90°C. for a period generally ranging from 3 to 24 hours and preferably from 5 to 10 hours. The quaternary ammonium reagent such as 2,3-epoxypropyltrialkylammoniumhalide or (chlorohydroxypropyl)trimethylammoniumhalide either in aqueous solution or crystalline form may be added either incrementally or all at once and stirred at a temperature of 40—90°C. and preferably at 60—70°C. for a period of about 4 hours or until the reaction is complete. These quaternary ammonium halide reagents are typically used in amounts of 0.003—0.95 mol per mol of hydroxyl and preferably from 0.1—0.3 mols per mol of hydroxyl group remaining unreacted on the polyvinyl alcohol base chain.

The reaction mixture is freed from all species below a certain molecular weight by dialysis or ultrafiltration. Two methods may be used, one a static and one a dynamic. In the static method the reaction mixture is placed inside a commercial semi-porous dialysis tube and the tubes are submerged in distilled water typically for periods of 12 to 48 hours. The contents of the tube are then recovered and the product may be used as is or dried. In the dynamic system a pressure pump is used to move water from the reaction mixture through a semi-porous membrane. The water carries out any inorganics and low molecular weight organics. The resulting concentrated product is then collected and may be used as is in cosmetic formulations or dried by conventional techniques to form a highly dispersible solid.

General Preparation VI (to produce a composition with a mixture of pendant groups)

A flask equipped with a water cooled condenser mechanical stirrer and thermometer is charged with polyvinyl alcohol and distilled water to form an aqueous suspension or slurry. The polyvinyl alcohol which is generally a commercially available product prepared by hydrolysis of polyvinyl acetate may have from 0—35% by weight residual acetate groups and preferably from 2—15% by weight. The number average molecular weight of the starting material may range from 2,000 up to 200,000 and higher and preferably from 25,000—150,000. In addition and for the purpose of this invention, a polyvinyl alcohol base chain may include up to 25% by weight of another comonomer such as vinylpyrrolidone, acrylic and methacrylic acid and esters thereof.

The aqueous slurry is heated to 80—100°C. and then a catalytic amount of an alkaline hydroxide such as sodium or potassium hydroxide or acidic material when appropriate as a catalyst is added. The entire solution is then generally treated with a quaternary ammonium reagent when used at a temperature of 40—90°C. for a period generally ranging from 3 to 24 hours and preferably from 5 to 10 hours. The quaternary ammonium reagent such as 2,3-epoxypropyltrialkylammoniumhalide or (chlorohydroxypropyl)trimethylammoniumhalide either in aqueous solution or crystalline form may be added either incrementally or all at once and stirred at a temperature of 40—90°C. and preferably at 60—70°C. for a period of about 4 hours or until the reaction is complete. These quaternary ammonium halide reagents are typically used in amounts of 0.003—0.95 mol per mol of hydroxyl and preferably from 0.1—0.3 mols per mol of hydroxyl group remaining unreacted on the polyvinyl alcohol base chain.

The reaction mixture is freed from all species below a certain molecular weight by dialysis or ultrafiltration. Two methods may be used, one a static and one a dynamic. In the static method the reaction mixture is placed inside a commercial semi-porous dialysis tube and the tubes are submerged in distilled water typically for periods of 12 to 48 hours. The contents of the tube are then recovered and the product may be used as is or dried. In the dynamic system a pressure pump is used to move water from the reaction mixture through a semi-porous membrane. The water carries out any inorganics and low molecular weight

organics. The resulting concentrated product is then collected and may be used as is in cosmetic formulations or may be then alkylated in the fashion described in the previous example.

The solution is then cooled to near ambient temperature if the addition of propylene oxide is intended or 50—75°C. when fatty epoxides or tertiary amine epoxides are to be added. These reagents may be used either singly or in combination in amounts ranging from 0—0.95 mol per mol of hydroxy groups on the polyvinyl alcohol chain and preferably from 0.001—0.5 mol per mol of hydroxyl on the base chain. A catalytic amount of alkaline hydroxide is used.

To obtain the optimum moisture barrier properties, the resulting solution is then treated by dialysis or ultrafiltration to remove the catalyst, affording an aqueous dispersion that may be used as is or recovered by precipitation.

The following example and preparations serve to illustrate the invention. All proportions used refer to parts by weight unless otherwise specified.

Preparation A (chlorohydroxypropyl)trimethylammonium chloride

A one-liter flask equipped with dropping funnel, mechanical stirrer, thermometer and condenser was charged with epichlorohydrin (95 grams, 1.02 mol) and placed in an isothermal bath at 18°C. Aqueous trimethylamine (238.7 grams, 25% by weight, 1.01 mol) was then added dropwise over a 3 hour period maintaining the temperature below 25°C. when the addition was complete the solution was allowed to stir overnight at ambient temperature.

Preparation B

$—RN^+R_1R_2R_3A^-=—CH_2—CH(OH)CH_2N(CH_3)_3Cl$

A flask equipped as described in General Preparation I was charged with 44 grams polyvinyl alcohol (commercially available) having a molecular weight of 126,000 (98% hydrolyzed—2% acetate) and 400 milliliters of distilled water. The resulting slurry was heated to 85°C. and held for 1 hour. The polymer dissolved completely to afford a pale yellow transparent solution. Potassium hydroxide (3.0 gram, 0.11 mol) in water (30 milliliters) was then added and the solution cooled to 60°C. Aqueous (chlorohydroxypropyl)trimethylammonium chloride (78 milliliters of 48% by weight aqueous solution prepared according to Preparation A), was then added all at once and the entire solution was heated to 60—65°C. for 4 additional hours. The warm solution was poured with stirring into acetone (2.0 liters) and allowed to stand. 63.5 Grams of colorless solid precipitate was collected by filtration shredded mechanically and dried under vacuum. The nitrogen content of the product was 2.15% by weight wherein the value for (m) in the above general formula segment is approximately 21.

Preparation C

Crystalline 2,3-epoxypropyltrimethylammonium chloride

A one-liter flask equipped with gas sparger, mechanical stirrer, thermometer and an aqueous acid trap was charged with epichlorohydrin (55.2 grams, 6.0 mol) and placed in an isothermal bath at 19°C. Trimethylamine gas (119.5 grams, 2.0 mol) was then sparged into the epichlorohydrin over a period of 3 hours. The temperature was maintained below 23°C. The solution was stirred for an additional 30 minutes and the precipitate collected by filtration. The crystalline product was washed with diethylether and vacuum dried to afford 262.2 grams of 2,3-epoxypropyltrimethylammonium chloride.

Preparation D

$—RN^+R_1R_2R_3A^-=—CH_2CH(OH)CH_2N(CH_3)_3Cl$

The procedure of Preparation B was repeated exactly except that pure crystalline 2,3-epoxypropyltrimethylammonium chloride (15.2 grams, 0.10 mol) was added in place of the aqueous solution. The mixture was then stirred for an additional 5.5 hours at 60°C. and recovered from acetone as described. The nitrogen content of the final product was 1.12% by weight wherein the value for (m) is approximately 28.

Preparation E

$—RN^+R_1R_2R_3A^-=—CH_2CH(OH)CH_2N(CH_3)_3Cl$

The procedure of Preparation B was reproduced except that the amount of aqueous 2,3-epoxypropyltrimethylammonium chloride was 56.2 milliliters of 48% solution and the warm aqueous polymer was recovered by precipitation in methanol (2.0 liters). The solid collected was shredded mechanically dried under vacuum and milled to a fine powder. The yield was 49.9 grams and the nitrogen content was 0.22% by weight wherein the value for (m) in the general formula segment is approximately 282.

Preparation F

$—RN^+R_1R_2R_3A^-=—CH_2CH(OH)CH_2N(CH_3)_3Cl$

The procedure of Preparation E was reproduced with the exception that the aqueous reaction mixture was cooled to ambient temperature and placed in a commercially available dialysis tube. The semi-pourous membrane in the dialysis tube retains all molecules with molecular weights greater than 8,000. The tubes were placed in water (4 liters) and allowed to stand. The bath water was changed ever 6—8

hours over a 36 hour period. The tubes were then recovered and the aqueous polymer solution inside was tested for moisture barrier properties.

Preparation G

$-RN^+R_1R_2R_3A^- = -CH_2CH(OH)CH_2N(CH_3)_3Cl$

The procedure of Preparation E was reproduced with the exception that recovery was accomplished by precipitation from acetone (1.5 liters). The scale was slightly reduced to 38.0 grams polyvinyl alcohol and 49 milliliters of 48% aqueous epoxide solution. The yield was 63.5 grams and the nitrogen content was 2.63% by weight wherein the value for (m) in the general formula segment is approximately 15.

Preparation H

Preparation of 2,3-epoxypropyltrimethylammonium bromide

A flask equipped with a sparger, mechanical stirrer, thermometer and efficiency condenser was charged with acetone (500 milliliters) at ambient temperature. Trimethylamine (58 grams, 0.98 mol) was charged into the solution. The pot was cooled to 10°C. and epibromohydrin (137 grams, 1.0 mol) was added dropwise over a 1 hour period. The resultant cloudy solution was allowed to slowly rise to ambient temperature and stand for 60 hours. Precipitate was collected by filtration washed with acetone (60 milliliters) and dried yielding a colorless crystalline product (179.4 grams, 94%).

Preparation I

$-RN^+R_1R_2R_3A^- = -CH_2CH(OH)CH_2N(CH_3)_3Br$

A flask equipped as described in General Preparation I was charged with polyvinyl alcohol (44.0 grams), (98% hydrolyzed, number average molecular weight equals 126,000) and 500 milliliters water. The slurry was warmed to 70°C. and potassium hydroxide (3.0 grams) was added. The pot was stirred an additional 30 minutes and 2,3-epoxypropyltrimethylammonium bromide (49.0 grams, 0.25 moles) was added through a powder funnel. This solution was stirred for 16 hours at 80°C. The solution was then poured into acetone (1500 milliliters) and the precipitate was collected by filtration, shredded, washed and dried. The pale brown solid powder which resulted had a nitrogen content of 2.10% by weight wherein the value for (m) in the general formula segment is approximately 19.

Preparation J

$-RN^+R_1R_2R_3A^- = -CH_2CH(OH)CH_2N^+(CH_3)_3Cl$

A flask equipped as described in General Preparation I was charged with polyvinyl alcohol (44.0 grams), (100% hydrolyzed, number average molecular weight equals 86,000), 200 milliliters distilled water and 3 grams potassium hydroxide. The pot was heated to 60°C. an aqueous (chlorohydroxypropyl)-trimethylammonium chloride (320 milliliters, 48% aqueous solution as prepared in Preparation A) was added. Heating was continued for an additional 24 hours and recovery was accomplished by precipitation from acetone. After collection washing and drying the nitrogen content was 0.72% wherein the value for (m) in the general formula segment is approximately 80.

Preparation K

$-RN^+R_1R_2R_3A^- = -CH_2CH(OH)CH_2N^+(CH_3)_3Cl$

A flask equipped as described in General Preparation I was charged with polyvinyl alcohol (88.0 grams), (98.5% hydrolyzed, having a molecular weight of 25,000) and 800 milliliters of water. The reaction was carried out exactly as described in Preparation D using crystalline 2,3-epoxypropyltrimethylammonium chloride (79.6 grams). Recovery was accomplished by precipitation in acetone. The nitrogen content was 1.01% by weight wherein the value for (m) in the general formula segment is approximately 84.

Preparation L

$-RN^+R_1R_2R_3A^- = -COCH_2N^+(CH_3)_2(CH_2Ph)Cl$

A flask equipped as described in General Preparation II was charged with polyvinyl alcohol (44.0 grams), (100% hydrolyzed, molecular weight 86,000), 500 milliliters dimethylformamide, 5.5 grams manganese acetate, and benzyldimethylethoxycarbonylmethyl ammonium chloride (51.0 grams, 0.25 mole). A nitrogen stream was passed through the solution as was heated to 125°C. and held for 6 hours. The resulting solution was poured into acetone (1500 milliliters) while hot. The precipitate was collected by filtration, shredded mechanically, washed again with polymer non-solvent and dried. The product was found to have a chloride content of 4.10% by weight and a calculated nitrogen content of 1.62% wherein the value for (m) in the general formula segment is approximately 28.

Preparation M

$-RN^+R_1R_2R_3A^- = -COCH_2N^+(CH_3)_2(CH_2Ph)Cl$

The process of Preparation L was repeated with polyvinyl alcohol (98% hydrolyzed having molecular weight of 126,000). The chloride content of the product was 4.41% by weight and the calculated nitrogen content was 1.72% wherein the value for (m) in the general formula segment is approximately 25.

Preparation N

$-RN^+R_1R_2R_3A^- = -CH_2CH(OH)CH_2N^+(CH_3)_3Cl$

Polyvinyl alcohol (44.0 g, MW=126,000, 98% hydrolyzed) was stirred in water (400 ml) and warmed to 85—90°C. The solution was cooled to 80°C. and aqueous potassium hydroxide (5.6 g in 30 ml $H_2O$) was added. This solution was cooled over 30 minutes to 60°C. and (3-chloro-2-hydroxypropyl)trimethylammonium chloride (18.8 g, 0.10 mol) was added all at once. The pH at this time was 7.3. The product was recovered by dialysis through a semi-porous membrane as previously described. The nitrogen content was 0.15% which corresponds to idealized formula with m=415.

Preparation O

$-RN^+R_1R_2R_3A^- = -CH_2CH(OH)CH_2N^+(CH_3)_3Cl$

Polyvinyl alcohol (22.0 g), (MW=126,000, 98% hydrolyzed) was stirred in water (210 ml) and warmed to 85°C. over 45 minutes. Potassium hydroxide (1.5 g in 10 ml $H_2O$) was then added and the solution cooled to 60°C. Crystalline 2,3-epoxypropyl-trimethylammonium chloride (75.8 g, 0.50 mol) was added and the entire solution held at 60°C. for 4 hours. The product was then recovered by precipitation from acetone, filtration and drying to afford 102 grams of white powder. The nitrogen content was 5.96% or 88% of theoretical, and wherein (m) has a value of approximately 2.0.

Preparation P

$-RN^+R_1R_2R_3A^- = -CH_2CH(OH)CH_2N^+(CH_3)_3Cl$

Polyvinyl alcohol (44.0 g), (MW=126,000, 98% hydrolyzed) was stirred in water (400 ml) and warmed to 85°C. over 30 minutes. The solution was cooled to 80°C. and Potassium hydroxide (3.0 g in 20 ml $H_2O$) was added. The entire solution was cooled to 60°C. over 45 minutes and 2,3-epoxypropyltrimethylammonium chloride (37.9 g in 100 ml $H_2O$) was added dropwise over 30 minutes. The solution was stirred at 60°C. for 3 additional hours and the product recovered by precipitation from acetone. The reaction afforded 50.4 grams of white solid with a nitrogen content of 2.92% or 71% of theoretical, and wherein (m) has a value of approximately 13.

Example 1

$-R_4 = -CH_2-CH(CH_3)OH;$

$-RN^+R_1R_2R_3A^- = -CH_2-CH(OH)CH_2N^+(CH_3)_3Cl$

A flask equipped with mechanical stirrer, thermometer, efficient condenser, and dropping funnel was charged with commercial polyvinyl alcohol (88.0 g) and distilled water (500 ml). The PVA contained 2% residual acetate groups and had a number average molecular weight of 126,000. The slurry was heated to 85—90°C. for one hour and potassium hydroxide (3.0 g in 15 ml $H_2O$) was added. The solution was cooled to 35°C and propylene oxide (35.0 g) was added dropwise at such a rate to prevent refluxing. When the addition was complete, the temperature was raised to 50°C. for 4 additional hours. The warm solution was then poured into acetone. The precipitate was collected by filtration, washed with acetone and methanol, and dried under vacuum. The yield was 113 g.

This propoxylated polyvinyl alcohol (56.5 g) was then charged to another flask equipped as described above along with water (500 ml), potassium hydroxide (3.0 g), and (chlorohydroxypropyl)trimethylammonium chloride 80 ml of 48% aqueous solution). The entire solution was then stirred at 60°C. for 16 hours. The product was recovered by precipitation from acetone described previously. The nitrogen content was found to be 0.88%.

Example 2

$-R_4 = -CH_2CH(OH)CH_3$

$-R-N^+R_1R_2R_3A^- = -CH_2CH(OH)CH_2-N(CH_3)_3Cl$

A flask equipped with thermometer, mechanical stirrer, condenser and nitrogen sparger was charged with polyvinyl alcohol (88.0 g, 98% hydrolyzed, MW=126,000) and distilled water (700 ml). Potassium hydroxide (3.0 g in 25 ml water) was added and the pot stirred at 35°C. for one hour. Propylene oxide (58.0 g, 1.0 mol) was then added dropwise. The exotherm was allowed to bring the pot temperature to 55°C. during the addition. When the addition was complete, the temperature was maintained at 55°C. for an additional 4 hours. Recovery was accomplished by precipitation in acetone as described previously. Yield was 134.5 g.

The propoxylated polyvinyl alcohol (72.2 g) was charged to a flask equipped as above along with distilled water (500 ml). The pot was warmed to 55°C. and potassium hydroxide (3.0 grams in 25 ml water) was added. After one hour, 2,3-epoxytrimethylammonium chloride (78 ml of 48% aqueous solution) was added in one slug and the entire solution warmed for an additional 12 hours. Recovery was accomplished by precipitation from acetone as described previously affording a product with a nitrogen content of 1.71%.

Example 3

$-R_4 = -CH[(CH_2)_7CH_3]CH(OH)(CH_2)_8OH$ or $-CH[(CH_2)_8OH]CH(OH)(CH_2)_7CH_3$

$-RN^+R_1R_2R_3A^- = -CH_2CH(OH)CH_2N(CH_3)_3Cl$

A two liter flask equipped with mechanical stirrer, thermometer and efficient condenser was charged

with oleyl alcohol (200 g, 85% purity) and methylene chloride (600 ml) and cooled to 5—10°C. 3-Chloroperoxybenzoic acid (135 g, 85% purity) was then added through a powder funnel in four equal portions spaced at 20 minutes intervals. The slurry was stirred an additional 3 hours allowing the temperature to rise gradually to ambient. The solution was then filtered and the filtrate extracted with 10% aqueous sodium bicarbonate (3×500 ml) and distilled water (2×500 ml). The organic layer was then collected, dried over anhydrous magnesium sulfate, and concentrated under vacuum to afford a pale yellow oil (212 g, 99% yield) which solidified to a colorless wax on standing. This wax was used without further purification.

A five liter, four necked flask equipped with thermometer, condenser and mechanical stirrer was charged with polyvinyl alcohol (500 g, 98% hydrolyzed, MW=126,000) and distilled water (3700 ml). The slurry was heated to 85°C. and held until dissolution was complete. Potassium hydroxide (31.5 g in 100 ml water) was added and the pot cooled to 70°C. 9,10-Epoxyoctadecan-1-ol (235 g, 0.83 mol) was then added and the pot cooled to 70°C. The opaque solution was stirred an additional 3 hours at 60°C. and allowed to cool gradually to ambient temperature.

The resulting latex-like solution was neutralized with a small amount of 6M $H_2SO_4$, and bottled. Half of this solution, measured volumetically, was then charged to similarly equipped three liter flask and warmed to 80°C. Potassium hydroxide (15.0 g in 50 ml $H_2O$) was added and the pot cooled to 60°C. Aqueous chlorohydroxypropyltrimethylammonium chloride (331.0 ml 48% aqueous solution) was then added all at once. The entire slurry was warmed at 60°C. for an additional 4 hours and recovered by precipitation from acetone.

Example 4

$—R_4=—CH[(CH_2)_7OH]CH(OH)(CH_2)_7CH_3$

$—RN^+R_1R_2R_3A^-=—CH_2—CH(OH)CH_2N^+(CH_3)_3Cl$

A flask equipped with a mechanical stirrer, thermometer, and condenser was charged with polyvinyl alcohol (44.0 g, 98% hydrolyzed, MW=126,000) and water (400 ml). The pot was heated to 85°C. for one hour and cooled to 80°C. Potassium hydroxide (3.0 g in 15 ml $H_2O$) was added and the pot was cooled to 60°C. Glycidyltrimethylammonium chloride (75.8 g) was then added, and the resultant solution stirred for 5 additional hours. The product was recovered by precipitation from acetone (1500 ml) as previously described. The yield was 110 grams and the nitrogen content was 4.66%.

This quaternized polymer (40.0 g) was then charged to a similarly equipped flask with water (400 ml). The pot was heated to 60—65°C. and potassium hydroxide (2.0 g in 5 ml $H_2O$) was added. 9,10-Epoxyoctadecanol (8.5 g), prepared as described in Example 3, was added and the solution was stirred at 60°C. for 16 hours. The product was then recovered from acetone and had a nitrogen content of 0.97%.

Example 5

$—R_4=—CH_2CH(OH)CH_3$

$—RN^+R_1R_2R_3A^-=—CH_2CH(OH)CH_2N^+(CH_3)_3Cl$

A flask equipped with a mechanical stirrer, condenser, thermometer, and dropping funnel was charged with 44.0 g polyvinyl alcohol (100% hydrolyzed, MW=78,000) and water (400 ml). The solution was warmed to 85—90°C. and sodium hydroxide (2.6 g in 20 ml $H_2O$) was added. The solution was then cooled to 30°C. Propylene oxide (15.0 g) was added all at once. The solution was stirred one hour at 30°C. and warmed to 70°C. 9,10-Epoxyoctadecanol (10.0 g) was then added along with glycidyltrimethylammonium chloride (30.0 g). The entire solution was then stirred at 70°C. for 3 hours, cooled to ambient temperature, and allowed to stand overnight. The product was recovered by precipitation in methanol.

Preparation Q

$—RNR_1R_2=—CH_2CH(OH)CH_2N(CH_3)(CH_2Ph)$

$—RN^+R_1R_2R_3A^-=—CH_2CH(OH)CH_2N^+(CH_3)_3Cl$

A flask equipped as described Preparation G was charged with polyvinyl alcohol (44.0 g) and water (400 ml) and heated to 85°C. After one hour, potassium hydroxide (3.1 g in 15 ml $H_2O$) was added and the pot cooled to 65°C. Glycidyltrimethylammonium chloride (30.0 g) was added all at once and the pot was stirred for one hour. Benzyl-(2,3-epoxypropyl)-methylamine was then added dropwise over a 30 minute period and the entire solution was stirred an additional 3 hours. The product was precipitated from methanol/acetone mixture to afford 62 g. The nitrogen content was 1.20%.

Preparation R

$—RNR_1R_2=—CH_2—CH(OH)CH_2N(CH_3)(CH_2Ph)$

A flask equipped with a mechanical stirrer, thermometer, dropping funnel and condenser was charged with polyvinyl alcohol (44.0 g) (98% hydrolyzed, MW=126,000) and water (400 ml). The slurry was warmed to 90°C. for one hour and potassium hydroxide (3.0 g in 15 ml $H_2O$) was added. The solution was cooled to 75°C. and freshly distilled benzyl-(2,3-epoxypropyl)-methylamine (44.3 g) was added dropwise over a 30 minute period. The entire solution was stirred 3 additional hours at 60—65°C. and recovered from acetone. The yield was 49.0 g and the nitrogen content was 0.25%.

Example 6

A flask equipped as described in the general example is charged with polyvinyl alcohol 44.0 g (MW=86,000, 100% hydrolyzed) and water (400 ml) and heated to 85—90°C. A catalytic amount of aqueous potassium hydroxide is added and the pot cooled to 70°C. Then 9,10-epoxyoctadecan-1-ol (14.2 g, 0.05 mol) is added and the entire slurry stirred for 3 hours, while gradually cooling to 60°C. Crystalline 2,3-epoxypropyltrimethylammonium chloride (151.5 g, 1.0 mol) is then added and the reaction mixture stirred 4 additional hours at 60°C. The product may then be recovered by precipitation or ultrafiltration to afford a product with a nitrogen content of 5.5% at an approximated yield of 84%.

Example 7

Another reaction done as described above except that the 2,3-epoxypropyltrimethylammonium chloride is reduced (75.8 g, 0.50 mol) should afford a product with a nitrogen content of 4.5% based on a reaction yield of 80%.

Example 8

A flask equipped as described in Preparation G may be charged with polyvinyl alcohol and distilled water. The aqueous slurry is heated to 80—90°C. and held for one hour or until the polymer is completely dispersed or solvated. A catalytic amount of an acid such as sulfuric acid or any proton acid or Lewis acid or aluminum hydrosilicate is added and the pot cooled to 40—90°C. At this point 9,10-epoxyoctadecanol is added and the reaction mixture is stirred for one hour. 2,3-Epoxypropyltrialkylammonium halide can then be added incrementally or all at once. These two epoxides combined may be used in 0.05—1.0 mol ratio preferably from 0.1—0.3 mol per mol of hydroxyl group on the polyvinyl alcohol base chain. The entire mixture is then stirred for an additional period typically 4 hours at 60°C. The product may then be recovered by one of the methods described.

A further example illustrates the effect of the salt formed by HCl neutralization on the skin moisture barrier properties. Note the substantial improvement of results based on the final dialyzed product.

Example 9

A flask equipped as described in the general example was charged with polyvinyl alcohol (44.0 g, MW=86,000, 100% hydrolyzed) and water (400 ml) and heated at 85—90°C. for one hour. The vessel was cooled to 80°C. and potassium hydroxide (5.6 g in 15 ml $H_2O$) is added. The vessel was cooled to 60°C. over one hour and 3-chloro-2-hydroxypropyltrimethyl ammonium chloride (18.8 g) was added. The solution was stirred four hours at 60°C. and dialyzed through a semi-porous membrane for five days. The nitrogen content was 0.15%. Improvement in product yields and a significant reduction in reaction time is achievable by using the oxirane derivative followed by addition to a concentrated PVA solution.

The solution was then placed in a flask equipped as described in the general example and heated to 80—85°C. Potassium hydroxide (0.56 g in 10 ml $H_2O$) was added and the solution stirred for one hour. While cooling to 60°C., 9,10-epoxyoctadecan-1-ol (10.2 g) was added and the entire solution was stirred at 60°C. for three hours. The resulting white dispersion was then split volumetrically into two aliquots. Solution A was neutralized with a small amount of concentrated HCl. This product was found to reduce the transpiration of water through the paper barrier by 42%. Solution B was dialyzed through a semi-porous membrane for 48 hours to pH=7.9. This product was found to reduce the transpiration of water through the paper barrier by 83%.

Moisture barrier test on paper

While the modified polyvinyl alcohol polymers of the invention are best tested for retentivity, substantivity and moisture barrier film forming properties on living animal skin an indication of their effectiveness as a moisture barrier can be obtained by testing on filter paper. In the test results listed in the following Table 1 a 2.5 inch (6,3 cm) circle of number 1 Wahtman filter paper was treated with aqueous solutions containing from 5—10% by weight of the modified polymer as described in the examples to obtain a polymer film deposit amounting to about 0.1 gram when dried at room temperature. The test is carried out by taking about 2 grams of aqueous solution of the modified polymer and dropping it over a water wet circle of filter paper from one side. The saturated paper is permitted to dry overnight at room temperature and weighed. Depending on the concentration of solution the procedure may be repeated until the weight pickup is about 0.1 gram so that each filter paper is treated with substantially an equal amount of polymer. The dry paper is sealed over the opening of a test cell containing 100 grams of water and permitted to stand for 100 hours in a constant humidity and temperature room at 70°F. (21.1°C.) at 40% relative humidity. The weight of water passing through the paper under these conditions is measured by weighing the amount of water remaining in the test cell. Each test employs a control cell containing the identical paper having no polymer treatment. Considering the weight loss through untreated paper as 100 the test results which are listed as percentage reduction in evaporation of water are calculated from the water remaining in the cell. The paper test results provide a rough indication of effectiveness as a moisture barrier for further testing on animal skin.

TABLE 1

| Example | % Reduction in evaporation |
|---|---|
| Control (unmodified PVA) | 40—50 |
| Preparation B | 55 |
| D | 36 |
| E | 61 |
| F | 58 |
| G | 33 |
| I | 59 |
| J | 46 |
| K | — |
| L | 40 |
| M | 51 |

In-vitro tests on animal skins having 1, 3 and 5 weight percent of the modified polyvinyl alcohol polymers of this invention indicate comparable results with regard to water vapor transmission on Neo-Natal Rat Stratum Corneum as shown in the results listed in Table 2.

TABLE 2
Water vapor transmission (5% polymer solution on neo-natal rat stratum corneum)

| Sample | % reduction |
|---|---|
| Control | 66.7 |
| Preparation B | 55.6 |
| Preparation J | 77.8 |
| Preparation K | 55.6 |

The effect of polymer coatings having 5% modified PVA polymers on the elasticity of pig skin is shown in Table 3.

TABLE 3
Elasticity of pig skin treated with 5% polymer solution

| Sample | Elasticity |
|---|---|
| Control | 7.8±2.6 |
| Preparation B | 8.8±2.0 |
| Preparation J | 11.4±2.0 |
| Preparation K | −1.6±0.8 |

Compares skin elasticity (petroleum jelly produces maximum elasticity—approximately 28 units).

The materials prepared according to Example 1 through 5 and Preparations Q and R when screened with the above described moisture barrier test on paper indicate a percent reduction in evaporation measuring from 35 to 50% which compare favorably with the 40—50% obtained with unmodified polyvinyl alcohol films. Of these, the product of Example 3 gave a result of 48% reduction.

In-vitro tests on animal skins having 5 weight percent of the modified polyvinyl alcohol polymers of this invention indicate comparable results with regard to water vapor transmission (WVT) on Neo-Natal Rat Stratum Corneum when compared with unmodified polyvinyl alcohol films. For example, when corneum membrane having a 5% film polymer as made according to Preparation E is tested, a 72.2% reduction in transmission is obtainable when compared with unmodified PVA (molecular weight of 126,000) having a 66.7% reduction.

Such films can be applied to skin surfaces without detrimental affect upon elasticity. For example, the composition of Example 3 when applied to pig skin at a 5% film loading and thereafter subjected to thermo-mechanical analysis provides a value of 11.4 while when compared with petroleum jelly which produces maximum elasticity gives a value of 28 units.

The polymers of this invention can be varied over a wide range to control the film forming properties and the formulation properties which are affected by the molecular weight of the PVA, the level of quaternary nitrogen, and the concentration of lipophilic groups attached to the PVA backbone. The exact hydrophile/lipophile ratio to achieve a balance of moisture retention, film properties, skin retention, wash off and formulation properties for these polymers is determined experimentally. In general those derivatized PVA polymers that contain high ratios of polyalkylene to quaternary groups in the side chain are

more lipophilic and less moisture sensitive, whereas, those compositions containing higher levels of nitrogen containing side groups tend to be more hydrophilic in character and lend themselves more readily for formulation in systems containing higher levels of water, glycerine or other hydrophilic molecules. There is no upper limit to the molecular weight of PVA that can be used to compare the polymers of this invention. In general it is found that above a molecular weight of several thousand, PVA film properties are sufficiently good to afford functional products. The film properties of PVA above 200,000 are excellent and may be used in the practice of this invention. However, practical limitations on availability of such starting materials and higher working viscosities make these materials somewhat less attractive.

Certain products of this invention may be polymeric surfactants in addition to moisture barriers and may be formulated in a variety of compositions with or without the addition of lower molecular weight surfactants. The choice of the surfactant depends on the specific formulation and properties desired and will depend on the molecular weight of the PVA, the level of quaternary nitrogen, the ratio of lipophilic side chain in the composition and the level of oil or water desired in the final formulation. With these experimental parameters and guidelines it is possible to prepare formulations which afford utilization of the polymers of this invention in a variety of applications including soap formulations, skin care products, vehicles for cosmetic formulations including pigments, powders, dyes, etc. useful in eye shadows, lotions and make-up. The products of this invention may also be used as vehicles for the inclusion of biocides, germicides, sunscreens and other biologically or chemically active molecules in film or particle form to protect burned skin from loss of moisture or infection.

The use of unmodified polyvinyl alcohol polymer in film forming ointment bases and barrier creams for use in protecting the skin against the action of external irritants has met with only limited success (J. B. Ward and G. J. Sperandio, "American Perfumer and Cosmetics" Volume 79, pages 53—55 (1964)). Film forming creams are difficult to produce with polyvinyl alcohol since they are either very difficult to formulate because of their poor mixing characteristics or they form poor films. Lotions and creams made with polyvinyl alcohol in general lack elegance, that is, the in-vitro films made from ointments and lotions containing about 15% polyvinyl alcohol are either slow drying, become greasy and tacky and eventually leave a film which is hard and leathery. Furthermore, good PVA moisture barrier films usually are hard to remove from the skin because they are difficult to remove with soap and water.

The problems associated with employing unmodified PVA in film forming bases are substantially overcome by the compositions of this invention in that they are easily dispersible in water are compatible with typical lotion formulations, and when applied to the surface of the skin, they dry quickly to form an elastic, smooth pellicle which retains its integrity over long period of time and is easily removed with soap and water. Tests for cosmetic elegance is accomplished by applying typical moisture barrier lotion formulas to the back of the hand and making observations with respect to ease of application, feel on the skin, time of drying, durability of the film, ease of removal and a host of subjective factors. In most instances, the formulations evaluated do not adversely effect the film forming characteristics of the modified polyvinyl alcohol compositions of this invention. The aqueous moisture barrier compositions of the invention generally have a lotion consistency and may be in the form of oil-in-water or water-in-oil emulsions with the former being preferred because of their more pleasing cosmetic properties. The lotions are preferably made by first preparing the oil phase then preparing the water phase and thereafter adding the water phase to the oil phase. Usually the aqueous phase materials are heated to a temperature of about 75 to about 100°C. and then added slowly with stirring to the oil phase which is heated to about the same temperature.

The oil phase components may contain a variety of material including emulsifiers, emollients, oils, waxes, perfumes, lanolins, polyalkylenes, stearols.

Water phase components may contain many different materials which include humectants, modified PVA moisture barrier components of the invention, proteins and polypeptides, preservatives, alkaline agents, thickening agents, perfumes, stabilizers and antiseptics.

The skin conditioning lotions and ointments of the invention contain as an essential ingredient from 0.1—15% by weight and preferably from 0.5—5% by weight of the above described modified polyvinyl alcohol polymers of the invention when used as cosmetics and pharmaceutical compositions. They may be added as aqueous compositions such as, in the case where the pendant groups are all of type (a) dispersions containing 0.1—30% by weight of modified polyvinyl alcohol or as dry powder.

The lotions may contain an emulsifier in an amount of from 0.05 to 8% and preferably from 0.25 to 5% to emulsify the oil components. Typical emulsifiers are selected from the group consisting of polyethoxylated fatty acids having less than about 30 mols of ethylene oxide per mol of fatty acid, ethyoxylated esters, unethoxylated sugar esters, polyoxyethylene fatty ether phosphates, fatty acid amides, phospholipids, polypropoxylated fatty ethers, acyllactates, polyethoxylated polyoxypropylene glycols, polypropoxylated polyoxyethylene glycols, polyoxyethylene, polyoxypropylene ethylene diamines, soaps and mixtures thereof.

Examples of such emulsifiers include polyoxyethylene (8) stearate, myristyl ethoxy (3) myristate, myristyl ethoxy (3) palmitate, methyl glucose sesquistearate, sucrose distearate, sucrose laurate, sorbitan monolaurate, polyoxyethylene (3) oleyl ether phosphate, polyoxyethylene (10) oleyl ether phosphate, lauric diethenyl amide, stearic monoethyl amide, lecithin, lanolin alcohol propoxylates, sodium stearoyl-2-lactate, calcium stearoyl-2-lactate, and the Pluronics® offered by BASF Wyandotte. Soaps such as alkaline

metal or triethanolamine salts of long chain fatty acids which include sodium stearate, triethanolamine stearate and similar salts of lanolin fatty acids. A preferred emulsifier is polyoxyethylene (21) stearyl ether.

The lotion formulations may contain an emollient material in an amount ranging from 0.2 to 25% and more often 1 to 8% by weight. One function of the emollient is to ensure that the modified polyvinyl alcohol polymer is classified sufficiently to allow it to be in a film-like state on the surface of the skin. Typical emollients are selected from the group consisting of fatty alcohols, esters having fewer than about 24 carbon atoms (for example, isopropylpalmitate), branch chain esters having greater than about 24 total carbon atoms (for example, cetearyl octonate), squalane, liquid or solid paraffins, mixtures of fatty acids and squalane, mixtures of fatty acids and liquid or solid paraffins and mixtures thereof. Typical alcohols and fatty acids which are useful include those having from 12 to 22 carbon atoms such as cetyl alcohol, myristyl alcohol, stearyl alcohol, stearic acid and palmitic acid. Paraffins include, for example, mineral oil, petrolatum and paraffin wax.

The lotions and ointments are particularly stable and effective when adjusted to a pH of 6—8.

Because of their high amine content some of the polymers of the invention may undergo decomposition in sunlight and air to form products which import an undesirable odor to the formulation. To overcome this there should be incorporated with the polymer a minor portion of a material which will inhibit the formation of such decomposition products. These inhibitors/antioxidant materials include nordihydrogucaretic acid, citric acid, ascorbic acid, hydroquinone, butylated hydroxy anisole, butylated hydroxytoluene, and any other suitable antioxidant.

The following formulations will serve to demonstrate but not limit the formulations containing the modified polyvinyl alcohol film forming moisture barrier polymer of the invention. Typical lotions contain 0.1—5.0% of the above described modified PVA polymers, 2—5% of an emollient such as a fatty alcohol, and 2—5% emulsifier in an aqueous emulsion.

Example A

Portions of the aqueous solution prepared according to Preparation F and Example 4 containing 0.5 grams of modified PVA polymer were diluted with water and added to an aqueous solution containing 2.4 grams cetyl alcohol, 1.6 grams stearyl alcohol and 3.0 grams of polyoxyethylene (21) stearyl ether (BRIJ® 721 surfactant by ICI Americans Inc.). Additional water was added to bring the water concentration to 92.5%. After stirring for about five minutes at 75°C. the emulsion is permitted to cool to room temperature and stored. The lotions were tested subjectively for cosmetic elegance by applying the product to the back of the hand and arm. They were determined to have smooth, silky feel, drying time of less than 15 minutes and a film durability in excess of two days. Residual films and lotions are easily removed from the skin with soap and water.

As mentioned above the polymers of the invention had advantageous cosmetic properties that permit them to be used in preparing cosmetic formulations either as ready to use compositions or concentrates which have to be diluted before use. Therefore, the cosmetic formula may contain the modified polyvinyl alcohol polymers in concentrations ranging from 0.01—15% by weight. The solution of these polymers are particularly useful when they are applied to hair, either alone or with other active substances during a treatment such as shampooing, dyeing, setting, blow drying, permanent waving, etc. They may improve notably the quality of the hair. When employed in hair treatment they facilitate untangling of wet hair and do not remain on dry hair as a sticky residue. In some instances they are expected to give dry hair additional life, a soft feel, a glossy appearance and resistance to tangling.

Hair treating formulations containing dilute aqueous, alcohol or dilute alcohol solutions of the modified polyvinyl alcohol polymer can be employed. Furthermore, they may be employed as creams, lotions, gels or as aerosol sprays. They may be used in combination with perfumes, dyes, preserving agents, sequestering agents, thickening agents, emulsifying agents.

Example B

Typical hair rinse formulations containing 5 grams of the modified polymers of Preparation K and Example 6, 7 grams cetyl alcohol, 3 grams of a linear polyoxyethylenated $C_{10}$—$C_{18}$ fatty alcohol, 2 grams of a casein derivative, 0.5 grams tetradecyltrimethylammonium chloride and 82.5 grams of water and a minor amount of hair dye can be used to treat hair having improved looks and anti-static properties.

Example C

A typical oxidation hair dye solution containing a 2.5 gram of the modified polymer of Preparation L or Example 7, 10 grams benzyl alcohol, 20 grams oleic acid, 3 grams polyoxyethylene (30), oleo cetyl alcohol, 7 grams oleic diethanolamide, 7.5 grams 2 octyldodecanol, 2.5 grams triethanolamine lauric sulfate, 10 grams ethanol, 18 milliliters aqueous ammonium, 1 gram n,n-bis(2-hydroxyethanol)paraphenylene-diamine, 0.4 grams resorcin, 0.15 grams m-aminophenol, .4 grams alphanaphthol, 0.1 grams hydroquinone. 0.24 grams ethylene diamine tetracetic acid, 1 milliliter sodium bisulfate, and water sufficient to make 100 grams is a typical ammonia oil composition for use as an oxidation hair dye when 130 grams of the solution is mixed with 30 grams of hydrogen peroxide bleach. After hair is treated with the material and allowed to stand for 30—40 minutes and thereafter rerinsed the hair is expected to untangle easily and have a silky touch.

The modified polyvinyl alcohol compositions of the invention may be employed to improve the elegance and stability of personal care products such as liquid and bar soaps, shaving creams, bath products, antiperspirants, sunscreens, cleansing creams and as a suspending agents for insoluble pigments and pharmaceutical actives. Improvement is generally realized when from 0.5—5% by weight of the compositions of this invention are employed in conventional formulations as hereinafter exemplified.

Example D

A portion of the aqueous solution prepared according to Example 3 containing 0.5 grams of fatty alcohol modified quaternized PVA was diluted with water and added to an aqueous solution containing 4.0 grams stearic acid, 2.0 grams polyoxyethylene (15) stearyl ether (Arlamol® E by ICI Americas Inc.), 5.0 grams glyceryl monostearate and polyoxyethylene stearate (Arlacel® 165 by ICI Americas Inc.), and 10.0 grams of 70% sorbitol solution (Sorbo® by ICI Americas Inc.). After stirring for about 5 minutes at 75°C. the emulsion is permitted to cool to room temperature and stored. The lotion was tested subjectively for cosmetic elegance by applying the product to the back of the hand and arm, was determined to have smooth, silky feel, drying time of less than 15 minutes and a film durability in excess of 2 days. Residual films and lotions are easily removed from the skin with soap and water.

Example E
Roll-on antiperspirant

| Ingredient | % W/W |
|---|---|
| Example 3 or Preparation E | 4.0 |
| polyoxyethylene (21) stearylether | 0.76 |
| polyoxyethylene (2) stearylether | 3.24 |
| water (deionized) | 34.76 |
| Dowicil 200®, Dow Chemical | 0.1 |
| Al Zr tetrachlorohydrex-Gly, Rezol 36G, Reheis | 57.14 |

Example F
Aerosol shave cream

| Ingredient | % W/W |
|---|---|
| Example 3 or Preparation E | 5.0 |
| Cetyl alcohol | 4.3 |
| polyoxyethylene (21) stearylether | 2.2 |
| sorbic acid | .17 |
| water | 74.9 |
| fragrance | .08 |
| water | 13.35 |

Example G
Oil-in-water sunscreen lotion

| Ingredient | W/W% |
|---|---|
| mineral oil | 18.8 |
| cetyl alcohol | 5.0 |
| Arlocel 60® emulsifier | 2.5 |
| Tween 60® emulsifier | 7.5 |
| Amyl para-dimethylaminobenzoic acid | 1.2 |
| Example 4 or Preparation F | 2.0 |
| water | 63.0 |
| Preservative | q.s. |

Example H
Water-in-oil pigmented makeup

| Ingredient | W/W% |
|---|---|
| Mineral oil | 10 |
| Beeswax | 1.5 |
| Cevesin wax | 1.0 |
| Arlacel 186® emulsifier | 3.2 |
| Sorbo® sorbitol | 28.8 |
| $TiO_2$ and other pigments | 20.0 |
| water | 33.5 |
| Preparation H or Q | 2.0 |

Example I
Calamine lotion

| | |
|---|---|
| Calamine | 80 gms |
| Zinc Oxide | 80 gms |
| glycerine | 20 mls |
| bentonite magma | 250 mls |
| calcium hydroxide (concentrated aqueous sol.) | 950 mls |
| Preparation B or P | 50 gms |

**Claims for the Contracting States: BE, CH, DE, FR, GB, IT, LI, LU, NL, SE**

1. A modified polyvinyl alcohol polymer which comprises a polyvinyl alcohol base chain having appendant thereto at least one oxygen-linked $R_4$ group and at least one further oxygen-linked pendant group selected from:

a) $—RN^{\oplus}R_1R_2R_3A^{\ominus}$

b) $RNR_1R_2$

wherein

R is selected from the group consisting of an alkylene, a substituted alkylene or acylene radical,

$R_1$, $R_2$, $R_3$ are alkyl or arylalkyl radicals having 1—20 carbon atoms,

$R_4$ is selected from the group consisting of alkyl, alkylaryl, substituted alkyl, alkylaryl, or a arylalkyl radicals bearing hydroxy or carboxyl groups or a combination of hydroxy and carboxyl groups,

$A^-$ is an anion, said polymer having a total nitrogen content ranging from 0.01—7.0% by weight.

2. A quaternary nitrogen-modified polyvinyl alcohol polymer according to claim 1, wherein said oxygen-linked pendant groups further comprises a mixture of $—RN^{\oplus}R_1R_2R_3A^{\ominus}$ groups and $—RNR_1R_2$ and $R_4$ groups wherein R, $R_1$, $R_2$, $R_3$, $R_4$ and A groups are as defined in claim 1.

3. A composition of either of the preceding claims, wherein said polyvinyl alcohol base chain has a number average molecular weight of at least 2,000.

4. A composition of any one of the preceding claims, wherein the cumulative formula weights of all $R_4$ groups are from 0.5—50% by weights of said polymer.

5. A composition of any one of the preceding claims, wherein the ratio of the total formula weights of all $—RNR_1R_2$ groups to the total formula weights of all of said $—RN^+R_1R_2R_3A^-$ groups range from 0—90% by weight.

6. A composition according to any one of the preceding claims, wherein R is a radical selected from the group consisting of

$$—CH_2—CHOH—CH_2— \quad \text{and} \quad —[CH_2CH(CH_2N^+R_1R_2R_3A^-)O]_eCH_2CH(OH)CH_2—$$

wherein $R_1$, $R_2$ and $R_3$ are alkyl or arylalkyl radicals having 1—20 carbon atoms, and $A^-$ is halide ion or (lower alkyl) $SO_4^-$ and e=1—20.

7. A composition of any one of the preceding claims, wherein said $RN^{\oplus}R_1R_2R_3A^{\ominus}$ group is $—COCH_2N^+R_1R_2R_3A^-$.

8. A composition according to any one of the preceding claims, wherein the pendant groups $R—NR_1R_2$ are selected from the group consisting of

$$—CH_2CHOH—CH_2NR_1R_2 \quad \text{and} \quad [—CH_2CH(CH_2NR_1R_2)—O]_eCH_2—CH(OH)—CH_2NR_1R_2$$

wherein $R_1$ and $R_2$ are alkyl or arylalkyl radicals having 1—20 carbon atoms and e=1—20.

9. A composition according to any one of the preceding claims, wherein the $—R—NR_1R_2$ pendant groups are $—COCH_2NR_1R_2$ wherein $R_1$ and $R_2$ are alkyl or arylalkyl radicals having 1—20 carbon atoms.

10. A composition according to any one of the preceding claims, where $R_1$, $R_2$ and $R_3$ are selected from the groups consisting of

$$-CH_3,\quad -CH_2CH_3,\quad -CH_2CH_2CH_3,\quad -\underset{CH_3}{\underset{|}{CH}}-CH_3,\quad \text{or}\quad -CH_2Ph$$

where Ph is phenyl.

11. A composition according to any one of the preceding claims, wherein the $-R_4$ is selected from the group consisting of 1,9-dihydroxy octadecane and 1,10-dihydroxy octadecane, octadecanoic acid, 9-hydroxy octadecanoic acid, 10 hydroxy octadecanoic acid, 9,10-dihydroxy octadecanoic acid and salts and esters of such acids.

12. A composition of claim 11, wherein one or both of the hydroxy groups of the 1,9 or 1,10-dihydroxy octadecane are oxy-linked to groups of the general formula $-R-NR_1R_2$ and/or $-R-N^+R_1R_2R_3A^-$ where R, $R_1$, $R_2$, $R_3$ and $A^-$ are the same as in claim 1.

13. A composition of claim 1, wherein $R_4$ is selected from the group consisting of

$$-\overset{O}{\overset{\|}{C}}-(CH_2)_{16}-CH_3,\quad -\overset{O}{\overset{\|}{C}}-(CH_2)_8-CH-(CH_2)_7-CH_3,\quad -\overset{O}{\overset{\|}{C}}-(CH_2)_7CH=CH-(CH_2)_7-CH_3,$$

and

$$-\overset{O}{\overset{\|}{C}}-(CH_2)_7\underset{OH}{\underset{|}{CH}}-\underset{OH}{\underset{|}{CH}}(CH_2)_7-CH_3.$$

14. A composition of claim 11, wherein part or all of the hydroxy groups on $R_4$ are oxy-linked to $-R-NR_1R_2$ and/or $-RN^+R_1R_2R_3A^-$ groups where R, $R_1$, $R_2$, $R_3$ and $A^-$ groups are the same as in claim 1.

15. A composition according to any one of the preceding claims, wherein said pendant groups comprise a mixture of at least two of $RN^{\oplus}R_1R_2R_3A^{\ominus}$ and $RNR_1R_2$ and $R_4$ groups where R, $R_1$, $R_2$, $R_3$, $R_4$ and $A^{\ominus}$ are as defined in claim 1, wherein $R_1$, $R_2$ and $R_3$ are methyl groups and the nitrogen content ranges from 0.05 to 3% by weight.

16. A composition according to claim 1, wherein the pendant groups comprise $RN^+R_1R_2R_3A^{\ominus}$ groups where $R_1$, $R_2$ and $R_3$ are methyl groups and the nitrogen content ranges from 0.01 to 3% by weight.

17. A composition of claim 1, when prepared by the steps of (a) reacting equal molar quantities of epichlorohydrin with aqueous trimethylamine at temperatures below 25°C., (b) forming an aqueous solution of polyvinyl alcohol having a number average molecular weight at least 2,000 containing catalytic amount of a base, (c) mixing the solutions of step (a) with step (b) and thereafter mixing as alkyl or substituted alkyl epoxide with the product of step (c).

18. A composition of claim 1, when prepared by the steps of: (a) reacting an alkyl or substituted alkyl epoxide with an aqueous solution of polyvinyl alcohol having a number average molecular weight in the range of 25,000—150,000 containing a catalytic amount of potassium hydroxide at a temperature of 30—90°C., (b) reacting epichlorohydrin with an aqueous solution of trimethylamine at a temperature below 25°C., (c) reacting the product of step (a) with product of step (b) at a temperature of 40—90°C.

19. A composition of claim 1, when prepared by the steps of: (a) adding (chlorohydroxypropyl)trialkylammonium chloride in aqueous or crystalline form to an aqueous solution of polyvinyl alcohol having a number average molecular weight of at least 2,000 in the presence of a catalytic amount of potassium hydroxide at a temperature of 40—90°C. and (b) reacting said reaction product of step (a) with an alkyl epoxide.

20. A composition of claim 1, when prepared by the steps of: (a) reacting an alkyl or substituted alkyl epoxide with aqueous polyvinyl alcohol having a number average molecular weight of 2,000—200,000 in the presence of a catalytic amount of potassium hydroxide at 30—90°C., and (b) reacting said reaction product of step (a) with (chlorohydroxypropyl) trimethylammonium chloride in either aqueous solution or crystalline form at a temperature of 40—90°C.

21. A composition of claim 1, when prepared by the steps of: (a) reacting in aqueous solution of polyvinyl alcohol having a number average molecular weight in the range of 25,000—150,000 with an aqueous solution of 2,3-epoxypropyltrialkylammonium halide in the presence of a catalytic amount of alkaline material at a temperature of 40—90°C. such that the mol ratio of said ammonium halide to each mol of hydroxy group on said polyvinyl alcohol polymer ranges from 0.003—1 and (b) reacting said product of step (a) with an alkyl epoxide.

22. A composition of claim 1, when prepared by the steps of: (a) reacting an alkyl or substituted alkyl epoxide with aqueous polyvinyl alcohol having a number average molecular weight in the range of 25,000—150,000 in the presence of catalytic amounts of potassium hydroxide at 30—90°C. until complete, and (b) reacting an aqueous solution of 2,3-epoxypropyltrialkylammonium halide with the product of step (a) at a temperature of 40—90°C. in the presence of a catalytic amount of base in amounts wherein the ratio

of said ammonium halide to each mol of hydroxyl group on said polyvinyl alcohol polymer ranges from 0.003—1.

23. A composition of claim 1, when prepared by the steps of: (a) reacting polyvinyl alcohol having a number of average molecular weight of 25,000—150,000 with trimethylethoxycarbonylmethylammonium halide in dipolar aprotic solution in the presence of catalytic amounts of magnesium acetate at a temperature of 150° and (b) reacting the product of step (a) with an alkyl or substituted alkyl epoxide.

24. A composition of claim 1, when prepared by the steps of: (a) reacting polyvinyl alcohol having a number average molecular weight of 25,000—150,000 with an alkyl epoxide in aqueous solution containing catalytic quantities of alkaline hydroxide, and (b) reacting the reaction product of step (a) with trimethylethoxycarbonylmethylammonium halide in dipolar aprotic solution in the presence of catalytic amounts of magnesium acetate at a temperature of 70—150°C.

25. A composition when prepared according to claim 19 or claim 22, wherein said ammonium halide is (chlorohydroxypropyl)-trimethylammonium chloride and said alkyl epoxide is 9,10-epoxy-octadecane-1-ol.

26. A composition when prepared according to claim 19 or 22, wherein said ammonium halide is 2,3-epoxypropyltrimethylammonium chloride and said alkyl epoxide is 9,10-epoxyoctadecane-1-ol.

27. A composition according to claim 1, wherein the product of any one of claims 17, 18, and 19 has been reacted with an alkyl epoxide at a temperature of 30—90°C.

28. A composition according to claim 1, wherein the product of claim 22 has been reacted with an alkyl or substituted alkyl epoxide in an aqueous reaction medium.

29. A composition of claim 1, when made by reacting an aqueous solution of polyvinyl alcohol polymer having a number average molecular weight of 25,000—150,000 with 9,10-epoxyoctadecanol and 2,3-epoxypropyltrialkylammonium halide at a temperature of 40—90°C. in the presence of a catalytic amount of acid wherein the combined mols of said epoxide to the mols of hydroxyl groups on the polyvinyl alcohol polymer ranges from 0.003—1.

30. A composition of claim 1, which is separated from an aqueous reaction mixture by precipitation through the addition of a nonsolvent selected from the group consisting of acetone and methanol.

31. A composition according to any one of the preceding claims, when purified in aqueous solution by dialysis or ultrafiltration.

32. An aqueous dispersion containing from 0.1—30% by weight of a composition according to any one of the preceding claims.

33. An aqueous skin conditioning composition comprising 0.1—5% by weight of a composition modified polyvinyl alcohol polymer comprising a polyvinyl alcohol base chain having oxygen-linked pendant groups selected from

a) $-RN^{\oplus}R_1R_2R_3A^{\ominus}$ and

b) a combination of $RNR_1R_2$ and $R_4$ wherein R, $R_1$, $R_2$, $R_3$, $R_4$ and A are as defined in claim 1, 2—5% by weight of an emulsifier and 2—5% by weight of an emollient.

34. An aqueous hair conditioning composition comprising 0.1—5% by weight of a modified polyvinyl alcohol polymer comprising a polyvinyl alcohol base chain having oxygen-linked pendant groups selected from

a) $-RN^{\oplus}R_1R_2R_3A^{\ominus}$ and

b) a combination of $RNR_1R_2$ and $R_4$ wherein R, $R_1$, $R_2$, $R_3$, $R_4$ and A are as defined in claim 1.

35. A cosmetic composition containing from 0.5—5% by weight of a modified polyvinyl alcohol polymer comprising a polyvinyl alcohol base chain having oxygen-linked pendant groups selected from

a) $-RN^{\oplus}R_1R_2R_3A^{\ominus}$ and

b) a combination of $RNR_1R_2$ and $R_4$ groups wherein R, $R_1$, $R_2$, $R_3$, $R_4$ and A are as defined in claim 1.

36. A pharmaceutical composition which comprises from 0.5—5% by weight of a modified polyvinyl alcohol polymer comprising a polyvinyl alcohol base chain having oxygen-linked pendant groups selected from

a) $-RN^{\oplus}R_1R_2R_3A^{\ominus}$ and

b) a combination of $RNR_1R_2$ and $R_4$ groups wherein R, $R_1$, $R_2$, $R_3$, $R_4$ and A are as defined in claim 1.

37. An amine modified polyvinyl alcohol polymer for use in skin conditioning, cosmetic and pharmaceutical formulations which comprises a polyvinyl base chain having a multiplicity of oxygen linked pendant groups of the general formula:

$$-RNR_1R_2$$

wherein

R is selected from a group consisting of an alkylene, a substituted alkylene or aceylene radical,

$R_1$ and $R_2$ are selected from the group consisting of alkyl or arylalkyl radicals having 1—20 carbon atoms, said polymer having a total nitrogen content ranging from 0.01—7% by weight.

38. A composition of any one of the preceding claims, wherein polyvinyl alcohol is a copolymer having up to 25% by weight of polyvinyl acetate, polyvinylacrylate, polyvinylmethacrylate and polyvinylpyrrolidone.

**Claims for the Contracting State: AT**

1. A process for preparing modified polyvinyl alcohol polymer which comprises a polyvinyl alcohol

base chain having appendant thereto at least one oxygen-linked $R_4$ group and at least one further oxygen-linked pendant group selected from:

a) $-RN^{\oplus}R_1R_2R_3A^{\ominus}$

b) $RNR_1R_2$

wherein

R is selected from the group consisting of an alkylene, a substituted alkylene or acylene radical,

$R_1$, $R_2$, $R_3$ are alkyl or arylalkyl radicals having 1—20 carbon atoms,

$R_4$ is selected from the group consisting of alkyl, alkylaryl, substituted alkyl, alkylaryl, or a arylalkyl radicals bearing hydroxy or carboxyl groups or a combination of hydroxy and carboxyl groups,

$A^-$ is an anion, said polymer having a total nitrogen content ranging from 0.01—7.0% by weight characterized by reacting poly vinyl alcohol with an $R_4$ group-containing reactant and at least one reactant containing an $-RN^{\oplus}R_1R_2R_3A^{\ominus}$ or $-RNR_1R_2$ group.

2. A process according to claim 1, wherein said oxygen-linked pendant groups further comprises a mixture of $-RN^{\oplus}R_1R_2R_3A^{\ominus}$ groups and $-RNR_1R_2$ and $R_4$ groups wherein R, $R_1$, $R_2$, $R_3$, $R_4$ and A groups are as defined in claim 1.

3. A process of either of the preceding claims, wherein said polyvinyl alcohol base chain has a number average molecular weight of at least 2,000.

4. A process of any one of the preceding claims, wherein the cumulative formula weights of all $R_4$ groups are from 0.5—50% by weights of said polymer.

5. A process of any one of the preceding claims, wherein the ratio of the total formula weights of all $-RNR_1R_2$ groups to the total formula weights of all of said $-RN^+R_1R_2R_3A^-$ groups range from 0—90% by weight.

6. A process according to any one of the preceding claims, wherein R is a radical selected from the group consisting of

$$-CH_2-CHOH-CH_2- \quad \text{and} \quad -[CH_2CH(CH_2N^+R_1R_2R_3A^-)O]_eCH_2CH(OH)CH_2-$$

wherein $R_1$, $R_2$ and $R_3$ are alkyl or arylalkyl radicals having 1—20 carbon atoms, and $A^-$ is halide ion or (lower alkyl) $SO_4^-$ and e=1—20.

7. A process of any one of the preceding claims, wherein said $RN^{\oplus}R_1R_2R_3A^{\ominus}$ group is $-COCH_2N^+R_1R_2R_3A^-$.

8. A process according to any one of the preceding claims, wherein the pendant groups $R-NR_1R_2$ are selected from the group consisting of

$$-CH_2CHOH-CH_2NR_1R_2 \quad \text{and} \quad [-CH_2CH(CH_2NR_1R_2)-O]_eCH_2-CH(OH)-CH_2NR_1R_2$$

wherein $R_1$ and $R_2$ are alkyl or arylalkyl radicals having 1—20 carbon atoms and e=1—20.

9. A process according to any one of the preceding claims, wherein the $-R-NR_1R_2$ pendant groups are $-COCH_2NR_1R_2$ wherein $R_1$ and $R_2$ are alkyl or arylalkyl radicals having 1—20 carbon atoms.

10. A process according to any one of the preceding claims, where $R_1$, $R_2$ and $R_3$ are selected from the groups consisting of

$$-CH_3, \quad -CH_2CH_3, \quad -CH_2CH_2CH_3, \quad -CH(CH_3)-CH_3, \quad \text{or} \quad -CH_2Ph$$

where Ph is phenyl.

11. A process according to any one of the preceding claims, wherein the $-R_4$ is selected from the group consisting of 1,9-dihydroxy octadecane and 1,10-dihydroxy octadecane, octadecanoic acid, 9-hydroxy octadecanoic acid, 10 hydroxy octadecanoic acid, 9,10-dihydroxy octadecanoic acid and salts and esters of such acids.

12. A process of claim 11, wherein one or both of the hydroxy groups of the 1,9 or 1,10-dihydroxy octadecane are oxy-linked to groups of the general formula $-R-NR_1R_2$ and/or $-R-N^+R_1R_2R_3A^-$ where R, $R_1$, $R_2$, $R_3$ and $A^-$ are the same as in claim 1.

13. A process of claim 1, wherein $R_4$ is selected from the group consisting of

$$-C(=O)-(CH_2)_{16}-CH_3, \quad -C(=O)-(CH_2)_8-CH-(CH_2)_7-CH_3, \quad -C(=O)-(CH_2)_7CH=CH-(CH_2)_7-CH_3,$$

and

$$-C(=O)-(CH_2)_7CH(OH)-CH(OH)(CH_2)_7-CH_3.$$

14. A process of claim 11, wherein part or all of the hydroxy groups on $R_4$ are oxy-linked to —R—$NR_1R_2$ and/or —$RN^+R_1R_2R_3A^-$ groups where R, $R_1$, $R_2$, $R_3$ and $A^-$ groups are the same as in claim 1.

15. A process according to any one of the preceding claims, wherein said pendant groups comprise a mixture of at least two of $RN^{\oplus}R_1R_2R_3A^{\ominus}$ and $RNR_1R_2$ and $R_4$ groups where R, $R_1$, $R_2$, $R_3$, $R_4$ and $A^{\ominus}$ are as defined in claim 1, wherein $R_1$, $R_2$ and $R_3$ are methyl groups and the nitrogen content ranges from 0.05 to 3% by weight.

16. A process according to claim 1, wherein the pendant groups comprise $RN^+R_1R_2R_3A^{\ominus}$ groups where $R_1$, $R_2$ and $R_3$ are methyl groups and the nitrogen content ranges from 0.01 to 3% by weight.

17. A process according to claim 1, comprising the steps of (a) reacting equal molar quantities of epichlorohydrin with aqueous trimethylamine at temperatures below 25°C., (b) forming an aqueous solution of polyvinyl alcohol having a number average molecular weight at least 2,000 containing catalytic amount of a base, (c) mixing the solutions of step (a) with step (b) and thereafter mixing as alkyl or substituted alkyl epoxide with the product of step (c).

18. A process according to claim 1, comprising the steps of: (a) reacting an alkyl or substituted alkyl epoxide with an aqueous solution of polyvinyl alcohol having a number average molecular weight in the range of 25,000—150,000 containing a catalytic amount of potassium hydroxide at a temperature of 30—90°C., (b) reacting epichlorohydrin with an aqueous solution of trimethylamine at a temperature below 25°C., (c) reacting the product of step (a) with product of step (b) at a temperature of 40—90°C.

19. A process according to claim 1, comprising the steps of: (a) adding (chlorohydroxypropyl)trialkylammonium chloride in aqueous or crystalline form to an aqueous solution of polyvinyl alcohol having a number average molecular weight of at least 2,000 in the presence of a catalytic amount of potassium hydroxide at a temperature of 40—90°C. and (b) reacting said reaction product of step (a) with an alkyl epoxide.

20. A process according to claim 1, comprising the steps of: (a) reacting an alkyl or substituted alkyl epoxide with aqueous polyvinyl alcohol having a number average molecular weight of 2,000—200,000 in the presence of a catalytic amount of potassium hydroxide at 30—90°C., and (b) reacting said reaction product of step (a) with (chlorohydroxypropyl) trimethylammonium chloride in either aqueous solution or crystalline form at a temperature of 40—90°C.

21. A process according to claim 1, comprising the steps of: (a) reacting in aqueous solution of polyvinyl alcohol having a number average molecular weight in the range of 25,000—150,000 with an aqueous solution of 2,3-epoxypropyltrialkylammonium halide in the presence of a catalytic amount of alkaline material at a temperature of 40—90°C. such that the mol ratio of said ammonium halide to each mol of hydroxyl group on said polyvinyl alcohol polymer ranges from 0.003—1 and (b) reacting said product of step (a) with an alkyl epoxide.

22. A process according to claim 1, comprising the steps of: (a) reacting an alkyl or substituted alkyl epoxide with aqueous polyvinyl alcohol having a number average molecular weight in the range of 25,000—150,000 in the presence of catalytic amounts of potassium hydroxide at 30—90°C. until complete, and (b) reacting an aqueous solution of 2,3-epoxypropyltrialkylammonium halide with the product of step (a) at a temperature of 40—90°C. in the presence of a catalytic amount of base in amounts wherein the ratio of said ammonium halide to each mol of hydroxyl group on said polyvinyl alcohol polymer ranges from 0.003—1.

23. A process according to claim 1, comprising the steps of: (a) reacting polyvinyl alcohol having a number average molecular weight of 25,000—150,000 with trimethylethoxycarbonylmethylammonium halide in dipolar aprotic solution in the presence of catalytic amounts of magnesium acetate at a temperature of 150° and (b) reacting the product of step (a) with an alkyl or substituted alkyl epoxide.

24. A process according to claim 1, comprising the steps of: (a) reacting polyvinyl alcohol having a number average molecular weight of 25,000—150,000 with an alkyl epoxide in aqueous solution containing catalytic quantities of alkaline hydroxide, and (b) reacting the reaction product of step (a) with trimethylethoxycarbonylmethylammonium halide in dipolar aprotic solution in the presence of catalytic amounts of magnesium acetate at a temperature of 70—150°C.

25. A process according to claim 19 or claim 22, wherein said ammonium halide is (chlorohydroxypropyl)trimethylammonium chloride and said alkyl epoxide is 9,10-epoxy-octadecane-1-ol.

26. A process according to claim 19 or 22, wherein said ammonium halide is 2,3-epoxypropyltrimethylammonium chloride and said alkyl epoxide is 9,10-epoxyoctadecane-1-ol.

27. A process according to claim 1, wherein the product of any one of claims 17, 18 and 19 has been reacted with an alkyl epoxide at a temperature of 30—90°C.

28. A process according to claim 1, wherein the product of claim 22 has been reacted with an alkyl or substituted alkyl epoxide in an aqueous reaction medium.

29. A process of claim 1, comprising reacting an aqueous solution of polyvinyl alcohol polymer having an number average molecular weight of 25,000—150,000 with 9,10-epoxyoctadecanol and 2,3-epoxypropyltrialkylammonium halide at a temperature of 40—90°C. in the presence of a catalytic amount of acid wherein the combined mols of said epoxide to the mols of hydroxyl groups on the polyvinyl alcohol polymer ranges from 0.003—1.

30. A process according to claim 1, wherein the desired composition is separated from an aqueous

reaction mixture by precipitation through the addition of a nonsolvent selected from the group consisting of acetone and methanol.

31. A process according to any one of the preceding claims, wherein the composition is purified in aqueous solution by dialysis or ultrafiltration.

32. An aqueous dispersion containing from 0.1—30% by weight of a composition according to any one of the preceding claims.

33. An aqueous skin conditioning composition comprising 0.1—5% by weight of a composition modified polyvinyl alcohol polymer comprising a polyvinyl alcohol base chain having oxygen-linked pendant groups selected from

a) $—RN^{\oplus}R_1R_2R_3A^{\ominus}$ and

b) a combination of $RNR_1R_2$ and $R_4$ wherein R is selected from the group consisting of an alkylene, a substituted alkylene or acylene radical, $R_1$, $R_2$, $R_3$ are alkyl or aryalkyl radicals having 1—20 carbon atoms, $R_4$ is selected from the group consisting of alkyl, alkylaryl, substituted alkyl, alkylaryl, or a arylalkyl radicals bearing hydroxy or carboxyl groups or a combination of hydroxy and carboxyl groups, $A^-$ is an anion, 2—5% by weight of an emulsifier and 2—5% by weight of an emollient.

34. An aqueous hair conditioning composition comprising 0.1—5% by weight of a modified polyvinyl alcohol polymer comprising a polyvinyl alcohol base chain having oxygen-linked pendant groups selected from

a) $—RN^{\oplus}R_1R_2R_3A^{\ominus}$ and

b) a combination of $RNR_1R_2$ and $R_4$ wherein R is selected from the group consisting of an alkylene, a substituted alkylene or acylene radical, $R_1$, $R_2$, $R_3$ are alkyl or aryalkyl radicals having 1—20 carbon atoms, $R_4$ is selected from the group consisting of alkyl, alkylaryl, substituted alkyl, alkylaryl, or a arylalkyl radicals bearing hydroxy or carboxyl groups or a combination of hydroxy and carboxyl groups, $A^-$ is an anion.

35. A cosmetic composition containing from 0.5—5% by weight of a modified polyvinyl alcohol polymer comprising a polyvinyl alcohol base chain having oxygen-linked pendant groups selected from

a) $—RN^{\oplus}R_1R_2R_3A^{\ominus}$ and

b) a combination of $RNR_1R_2$ and $R_4$ groups wherein R is selected from the group consisting of an alkylene, a substituted alkylene or acylene radical, $R_1$, $R_2$, $R_3$ are alkyl or aryalkyl radicals having 1—20 carbon atoms, $R_4$ is selected from the group consisting of alkyl, alkylaryl, substituted alkyl, alkylaryl, or a arylalkyl radicals bearing hydroxy or carboxyl groups or a combination of hydroxy and carboxyl groups, $A^-$ is an anion.

36. A composition of any one of claims 32 to 35, wherein the polyvinyl alcohol is a copolymer having up to 25% by weight of polyvinyl acetate, polyvinylacrylate, polyvinylmethacrylate and polyvinylpyrrolidone.

**Patentansprüche für die Vertragsstaaten: BE, CH, DE, FR, GB, IT, LI, LU, NL, SE**

1. Modifiziertes Polyvinylalkoholpolymer mit einer Polyvinylalkohol-Hauptkette, an der mindestens eine mit Sauerstoff verbundene Gruppe $R_4$ hängt, sowie mindestens eine weitere mit Sauerstoff verbundene Gruppe, die ausgewählt ist aus

a) $—RN^{\oplus}R_1R_2R_3A^{\ominus}$

b) $RNR_1R_2$

wobei

R aus der Gruppe ausgewählt ist, die aus einem Alkylen-, einem substituierten Alkylen- oder einem Acylenradikal besteht,

$R_1$, $R_2$, $R_3$ Alkyl- oder Arylalkylradikale mit 1 bis 20 Kohlenstoffatomen sind,

$R_4$ aus der Gruppe ausgewählt ist, die aus den Alkyl-, Alkylaryl-, substituierten Alkyl-, Alkylaryl- oder Arylalkylradikalen besteht, die Hydroxy- oder Carboxylgruppen oder eine Kombination von Hydroxy- und Carboxylgruppen tragen, und

$A^-$ ein Anion ist,

wobei der Gesamtstickstoffgehalt des Polymers im Bereich von 0,01 bis 7,0% liegt.

2. Quaternäres stickstoffmodifiziertes Polyvinylalkoholpolymer nach Anspruch 1, dadurch gekennzeichnet, daß die mit Sauerstoff verbundenen angehängten Gruppen mindestens teilweise aus einem Gemisch von Gruppen $—RN^{\oplus}R_1R_2R_3A^{\ominus}$ und von Gruppen $—RNR_1R_2$ und $R_4$ bestehen, wobei die Gruppen R, $R_1$, $R_2$, $R_3$, $R_4$ und A wie im Anspruch 1 definiert sind.

3. Zusammensetzung nach einem der vorhergehenden Ansprüche, dadurch gekennzeichnet, daß die Polyvinylalkohol-Hauptkette ein zahlenmäßig durchschnittliches Molekulargewicht von mindestens 2000 hat.

4. Zusammensetzung nach einem der vorhergehenden Ansprüche, dadurch gekennzeichnet, daß das Gesamtformelgewicht aller Gruppen $R_4$ 0,5 bis 50 Gewichtsprozent des Polymers beträgt.

5. Zusammensetzung nach einem der vorhergehenden Ansprüche, dadurch gekennzeichnet, daß das Verhältnis des Gesamtformelgewichts aller Gruppen $—RNR_1R_2$ zu dem Gesamtformelgewicht aller Gruppen $RN^+R_1R_2R_3A^-$ im Bereich von 0 bis 90 Gew.% liegt.

6. Zusammensetzung nach einem der vorhergehenden Ansprüche, dadurch gekennzeichnet, daß R ein Radikal ist, das aus der Gruppe ausgewählt ist, die aus

$$—CH_2—CHOH—CH_2 \quad \text{und} \quad —[CH_2CH(CH_2N^+R_1R_2R_3A^-)O]_eCH_2CH(OH)CH_2—$$

besteht, wobei $R_1$, $R_2$ und $R_3$ Alkyl- oder Aralkylradikale mit 1 bis 20 Kohlenstoffatomen sind und $A^-$ ein Halogenidion oder ein (niederes Alkyl)—$SO_4^-$ und e=1 bis 20 ist.

7. Zusammensetzung nach einem der vorhergehenden Ansprüche, dadurch gekennzeichnet, daß die Gruppe $RN^{\oplus}R_1R_2R_3A^{\ominus}$ die Gruppe $-COCH_2N^+R_1R_2R_3A^-$ ist.

8. Zusammensetzung nach einem der vorhergehenden Ansprüche, dadurch gekennzeichnet, daß die angehängten Gruppen $R-NR_1R_2$ aus der Gruppe ausgewählt sind, die aus

$$-CH_2CHOH-CH_2NR_1R_2 \quad \text{und} \quad [-CH_2CH(CH_2NR_1R_2)-O]_eCH_2-CH(OH)-CH_2NR_1R_2$$

besteht, wobei R und R Alkyl- oder Aralkylradikale mit 1 bis 20 Kohlenstoffatomen sind und e=1 bis 20 ist.

9. Zusammensetzung nach einem der vorhergehenden Ansprüche, dadurch gekennzeichnet, daß die angehängten Gruppen $-R-NR_1R_2$ Gruppen $-COCH_2NR_1R_2$ sind, in denen $R_1$ und $R_2$ Alkyl- oder Arylalkylradikale mit 1 bis 20 Kohlenstoffatomen sind.

10. Zusammensetzung nach einem der vorhergehenden Ansprüche, dadurch gekennzeichnet, daß $R_1$, $R_2$ und $R_3$ aus der Gruppe ausgewählt sind, die aus

$$-CH_3,\quad -CH_2CH_3,\quad -CH_2CH_2CH_3,\quad -\underset{CH_3}{\underset{|}{CH}}-CH_3,\quad \text{und} \quad -CH_2Ph$$

besteht, wobei Ph Phenyl ist.

11. Zusammensetzung nach einem der vorhergehenden Ansprüche, dadurch gekennzeichnet, daß $-R_4$ aus der Gruppe ausgewählt ist, die aus 1,9-Dihydroxyoctadecan und 1,10-Dihydroxyoctadecan, Octadecansäure, 9-Hydroxyoctadecansäure, 10-Hydroxyoctadecansäure, 9,10-Dihydroxyoctadecansäure und den Salzen und Estern dieser Säuren besteht.

12. Zusammensetzung nach Anspruch 11, dadurch gekennzeichnet, daß eine oder beide der Hydroxygruppen des 1,9- oder 1,10-Dihydroxyoctadecans durch Sauerstoff mit Gruppen der allgemeinen Formel $-R-NR_1R_2$ und/oder $-R-N^+R_1R_2R_3A^-$ verbunden sind, in denen R, $R_1$, $R_2$, $R_3$ und $A^-$ dieselbe Bedeutung haben wie im Anspruch 1.

13. Zusammensetzung nach Anspruch 1, dadurch gekennzeichnet, daß $R_4$ aus der Gruppe ausgewählt ist, die aus

$$-\overset{O}{\overset{\|}{C}}-(CH_2)_{16}-CH_3,\quad -\overset{O}{\overset{\|}{C}}-(CH_2)_8-CH-(CH_2)_7-CH_3,\quad -\overset{O}{\overset{\|}{C}}-(CH_2)_7CH-CH-(CH_2)_7-CH_3,$$

und

$$-\overset{O}{\overset{\|}{C}}-(CH_2)_7\underset{OH}{\underset{|}{CH}}-\underset{OH}{\underset{|}{CH}}(CH_2)_7-CH_3$$

besteht.

14. Zusammensetzung nach Anspruch 11, dadurch gekennzeichnet, daß ein Teil oder alle der Hydroxygruppen von $R_4$ durch Sauerstoff mit Gruppen $-R-NR_1R_2$ und/oder $-R-N^+R_1R_2R_3A^-$ verbunden sind, wobei die Gruppen R, $R_1$, $R_2$, $R_3$ und $A^-$ dieselben sind wie im Anspruch 1.

15. Zusammensetzung nach einem der vorhergehenden Ansprüche, dadurch gekennzeichnet, daß die angehängten Gruppen mindestens teilweise aus einem Gemisch aus mindestens zwei Gruppen $RN^{\oplus}R_1R_2R_3A^{\ominus}$, $RNR_1R_2$ und $R_4$ bestehen, in denen R, $R_1$, $R_2$, $R_3$, $R_4$ und $A^{\ominus}$ die im Anspruch 1 angegebenen Bedeutungen haben, $R_1$, $R_2$ und $R_3$ Methylgruppen sind und der Sticksstoffgehalt im Bereich von 0,05 bis 3 Gew.% beträgt.

16. Zusammensetzung nach Anspruch 1, dadurch gekennzeichnet, daß die angehängten Gruppen mindestens teilweise aus Gruppen $RN^+R_1R_2R_3A^{\ominus}$ bestehen, in denen $R_1$, $R_2$ und $R_3$ Methylgruppen sind und der Stickstoffgehalt im Bereich von 0,01 bis 3 Gew.% liegt.

17. Zusammensetzung nach Anspruch 1, zu deren Erzeugung folgende Schritte durchgeführt wurden: (a) äquimolare Mengen Epichlorhydrin und wäßriges Trimethylamin werden bei Temperaturen unter 25°C umgesetzt; (b) es wird eine wäßrige Lösung von Polyvinylalkohol mit einem zahlenmäßig durchschnittlichen Molekulargewicht von mindestens 2000 hergestellt, die eine Base in einer katalytisch wirksamen Menge enthält, (c) die in den Schritten (a) und (b) hergestellten Lösungen werden miteinander vermischt, und danach wird ein Alkyl- oder substituiertes Alkylepoxid mit dem Produkt des Schritts (c) vermischt.

18. Zusammensetzung nach Anspruch 1, die durch folgende Schritte durchgeführt worden ist: (a) ein Alkyl- oder substituiertes Alkylepoxid wird bei einer Temperatur von 30 bis 90°C mit einer wäßrigen Lösung eines Polyvinylalkohols umgesetzt, der ein zahlenmäßig durchschnittliches Molekulargewicht im Bereich von 25 000 bis 150 000 hat, wobei die Lösung Kaliumhydroxid in einer katalytisch wirksamen Menge enthält; (b) Epichlorhydrin und eine wäßrige Lösung von Trimethylamin werden bei einer Temperatur

unter 25°C umgesetzt, (c) die in den Schritten (a) und (b) erhaltenen Produkte werden bei einer Temperatur von 40 bis 90°C umgesetzt.

19. Zusammensetzung nach Anspruch 1, die durch folgende Schritte erzeugt worden ist: (a) (Chlorhydroxypropyl)trialkylammoniumchlorid in wäßriger oder kristalliner Form wird bei einer Temperatur von 40 bis 90°C mit einer wäßrigen Lösung eines Polyvinylalkohols mit einem zahlenmäßig durchschnittlichen Molekulargewicht von mindestens 2000 in Gegenwart einer katalytisch wirksamen Menge Kaliumhydroxid umgesetzt; und (b) das im Schritt (a) erhaltene Reaktionsprodukt wird mit einem Alkylepoxid umgesetzt.

20. Zusammensetzung nach Anspruch 1, die durch folgende Schritte erzeugt worden ist: (a) ein Alkyl- oder substituiertes Alkylepoxid wird bei 30 bis 90°C in Gegenwart einer katalytisch wirksamen Menge Kaliumhydroxid mit einem wäßrigen Polyvinylalkohol umgesetzt, der ein zahlenmäßig durchschnittliches Molekulargewicht von 2000 bis 200 000 hat; (b) das im Schritt (a) erhaltene Reaktionsprodukt wird bei einer Temperatur von 40 bis 90°C mit (Chlorhydroxypropyl)trimethylammoniumchlorid in Form einer wäßrigen Lösung oder kristalliner Form umgesetzt.

21. Zusammensetzung nach Anspruch 1, die durch folgende Schritte erzeugt worden ist: (a) in wäßriger Lösung wird ein Polyvinylalkohol mit einem zahlenmäßig durchschnittlichen Molekulargewicht im Bereich von 25 000 bis 150 000 bei einer Temperatur von 40 bis 90°C mit einer wäßrigen Lösung von 2,3-Epoxypropyltrialkylammoniumhalogenid in Gegenwart einer katalytisch wirksamen Menge einer alkalischen Substanz umgesetzt, wobei das Verhältnis der Molanzahl des Ammoniumhalogenids zu der Molanzahl der Hydroxylgruppe des Polyvinylalkoholpolymers im Bereich von 0,003 bis 1 liegt; (b) das im Schritt (a) erhaltene Produkt wird mit einem Alkylepoxid umgesetzt.

22. Zusammensetzung nach Anspruch 1, die durch folgende Schritte erzeugt worden ist: (a) ein Alkyl- oder substituiertes Alkylepoxid wird bei 30 bis 90°C in Gegenwart einer katalytisch wirksamen Menge Kaliumhydroxid mit einem wäßrigen Polyvinylalkohol mit einem zahlenmäßig durchschnittlichen Molekulargewicht im Bereich von 25 000 bis 150 000 umgesetzt, bis die Reaktion vollständig durchgeführt worden ist; (b) eine wäßrige Lösung von 2,3-Epoxypropyltrialkylammoniumhalogenid wird bei einer Temperatur von 40 bis 90°C mit dem im Schritt (a) erhaltenen Produkt in Gegenwart einer katalytisch wirksamen Menge einer Base umgesetzt, und zwar in solchen Mengen, daß das Verhältnis der Molanzahl des Ammoniumhalogenids zu der Molanzahl der Hydroxylgruppen des Polyvinylalkoholpolymers im Bereich von 0,003 bis 1 liegt.

23. Zusammensetzung nach Anspruch 1, die durch folgende Schritte erzeugt worden ist: (a) ein Polyvinylalkohol mit einem zahlenmäßig durchschnittlichen Molekulargewicht von 25 000 bis 150 000 wird bei einer Temperatur von 150°C mit einem Trimethylethoxycarbonylmethylammoniumhalogenid in einer dipolaren aprotischen Lösung in Gegenwart einer katalytisch wirksamen Menge Magnesiumacetat umgesetzt; (b) das im Schritt (a) erhaltene Produkt wird mit einem Alkyl- oder einem substituierten Alkylepoxid umgesetzt.

24. Zusammensetzung nach Anspruch 1, die durch folgende Schritte erzeugt worden ist: (a) ein Polyvinylalkohol mit einem zahlenmäßig durchschnittlichen Molekulargewicht von 25 000 bis 150 000 wird mit einem Alkylepoxid in einer wäßrigen Lösung umgesetzt, die eine katalytisch wirksame Menge Alkalihydroxid enthält; (b) das im Schritt (a) erhaltene Reaktionsprodukt wird bei einer Temperatur von 70 bis 150°C in Gegenwart einer katalytisch wirksamen Menge Magnesiumacetat mit Trimethylethoxycarbonylmethylammoniumhalogenid in einer dipolaren aprotischen Lösung umgesetzt.

25. Zusammensetzung, die nach Anspruch 19 oder 22 erzeugt worden ist, wobei das Ammoniumhalogenid (Chlorohydroxypropyl)-Trimethylammoniumchlorid und das Alkylepoxid 9,10-Epoxyoctadecan-1-ol ist.

26. Zusammensetzung, die nach Anspruch 19 oder 22 erzeugt worden ist, wobei das Ammoniumhalogenid 2,3-Epoxypropyltrimethylammoniumchlorid und das Alkylepoxid 9,10-Epoxyoctadecan-1-ol ist.

27. Zusammensetzung nach Anspruch 1, dadurch gekennzeichnet, daß das Produkt nach einem der Ansprüche 17, 18 und 19 bei einer Temperatur von 30 bis 90°C mit einem Alkylepoxid umgesetzt worden ist.

28. Zusammensetzung nach Anspruch 1, dadurch gekennzeichnet, daß das Produkt nach Anspruch 25 in einem wäßrigen Reaktionsmedium mit einem Alkyl- oder substituierten Alkylepoxid umgesetzt worden ist.

29. Zusammensetzung nach Anspruch 1, zu deren Erzeugung eine wäßrige Lösung eines Polyvinylalkoholpolymers mit einem zahlenmäßig durchschnittlichen Molekulargewicht von 25 000 bis 150 000 bei einer Temperatur von 40 bis 90°C in Gegenwart einer katalytisch wirksamen Menge Säure mit 9,10-Epoxyoctadecanol und 2,3-Epoxypropyltrialkylammoniumhalogenid umgesetzt worden ist, wobei das Verhältnis der Gesamtanzahl der Mole des Epoxid zu der Anzahl der Mole der Hydroxygruppen des Polyvinylalkoholpolymers im Bereich von 0,003 bis 1 liegt.

30. Zusammensetzung nach Anspruch 1, die aus einem wäßrigen Reaktionsgemisch durch Zusatz eines Nichtlösungsmittels ausgefällt worden ist, das aus der Gruppe ausgewählt ist, die aus Aceton und Methanol besteht.

31. Zusammensetzung nach einem der vorhergehenden Ansprüche, die in wäßriger Lösung durch Dialyse oder Ultrafiltration gereinigt worden ist.

32. Wäßrige Dispersion, die 0,1 bis 30 Gew.% einer Zusammensetzung nach einem der vorhergehenden Ansprüche enthält.

33. Wäßrige Zusammensetzung zum Konditionieren der Haut, die 0,1 bis 5 Gew.% einer Zusammensetzung enthält, die ein modifiziertes Polyvinylalkoholpolymer enthält, das eine Polyvinylalkohol-Hauptkette besitzt, an die mit Sauerstoff verbundene Gruppen angehängt sind, die ausgewählt sind aus

a) $-RN^{\oplus}R_1R_2R_3A^{\ominus}$ und

b) einer Kombination von $RNR_1R_2$ und $R_4$,

wobei R, $R_1$, $R_2$, $R_3$, $R_4$ und A die im Anspruch 1 angegebenen Bedeutungen haben, ferner 2 bis 5 Gew.% eines Emulgators und 2 bis 5 Gew.% eines Erweichungsmittels.

34. Wäßrige Zusammensetzung zum Konditionieren der Haare, die 0,1 bis 5 Gew.% einer Zusammensetzung enthält, die ein modifiziertes Polyvinylalkoholpolymer enthält, das eine Polyvinylalkohol-Hauptkette besitzt, an die mit Sauerstoff verbundene Gruppen angehängt sind, die ausgewählt sind aus

a) $-RN^{\oplus}R_1R_2R_3A^{\ominus}$ und

b) einer Kombination von $RNR_1R_2$ und $R_4$,

wobei R, $R_1$, $R_2$, $R_3$, $R_4$ und A die im Anspruch 1 angegebenen Bedeutungen haben.

35. Kosmetische Zusammensetzung, die 0,5 bis 5 Gew.% einer Zusammensetzung enthält, die ein modifiziertes Polyvinylalkoholpolymer enthält, das eine Polyvinylalkohol-Hauptkette besitzt, and die mit Sauerstoff verbundene Gruppen angehängt sind, die ausgewählt sind aus

a) $-RN^{+}R_1R_2R_3A^{-}$ und

b) einer Kombination von Gruppen $RNR_1R_2$ und $R_4$,

wobei R, $R_1$, $R_2$, $R_3$, $R_4$ und A die im Anspruch 1 angegebenen Bedeutungen haben.

36. Pharmazeutische Zusammensetzung, die 0,5 bis 5 Gew.% einer Zusammensetzung enthält, die ein modifiziertes Polyvinylalkoholpolymer enthält, das eine Polyvinylalkohol-Hauptkette besitzt, an die mit Sauerstoff verbundene Gruppen angehängt sind, die ausgewählt sind aus

a) $-RN^{\oplus}R_1R_2R_3A^{\ominus}$ und

b) einer Kombination von Gruppen $RNR_1R_2$ und $R_4$,

wobei R, $R_1$, $R_2$, $R_3$, $R_4$ und A die im Anspruch 1 angegebenen Bedeutungen haben.

37. Aminmodifiziertes Polyvinylalkoholpolymer für die Verwendung in Hautkonditionier-, kosmetischen und pharmazeutischen Präparaten, mit einer Polyvinylhauptkette mit einer Vielzahl von mit Sauerstoff verbundenen, angehängten Gruppen der allgemeinen Formel

$$-RNR_1R_2$$

in der

R aus der Gruppe ausgewählt sit, die aus einem Alkylen-, einem substituierten Alkylen- und einem Acetylenradikal besteht und

$R_1$ und $R_2$ aus der Gruppe ausgewählt sind, die aus den Alkyl- und den Arylalkylradikalen mit 1 bis 20 Kohlenstoffatomen besteht,

wobei das Polymer einen Gesamtstickstoffgehalt von 0,01 bis 7 Gew.% besitzt.

38. Zusammensetzung nach einem der vorhergehenden Ansprüche, dadurch gekennzeichnet, daß das Polyvinylalkoholpolymer ein Copolymer ist, das bis zu 25 Gew.% Polyvinylacetat, Polyvinylacrylat, Polyvinylmethacrylat und Polyvinylpyrrolidon enthält.

**Patentansprüche für den Vertragsstaat: AT**

1. Verfahren zum Erzeugen eines modifizierten Polyvinylalkoholpolymers mit einer Polyvinylalkohol-Hauptkette, and der mindestens eine mit Sauerstoff verbundene Gruppe $R_4$ hängt, sowie mindestens eine weitere mit Sauerstoff verbundene Gruppe, die ausgewählt ist aus

a) $-RN^{\oplus}R_1R_2R_3A^{\ominus}$

b) $RNR_1R_2$

wobei

R aus der Gruppe ausgewählt ist, die aus einem Alkylen-, einem substituierten Alkylen- oder einem Acylenradikal besteht,

$R_1$, $R_2$, $R_3$ Alkyl- oder Arylalkylradikale mit 1 bis 20 Kohlenstoffatomen sind,

$R_4$ aus der Gruppe ausgewählt ist, die aus den Alkyl-, Alkylaryl-, substituierten Alkyl-, Alkylaryl- oder Arylalkylradikalen besteht, die Hydroxy- oder Carboxylgruppen oder eine Kombination von Hydroxy- und Carboxylgruppen tragen, und

$A^{-}$ ein Anion ist,

wobei der Gesamtstickstoffgehalt des Polymers im Bereich von 0,01 bis 7,0% liegt, dadurch gekennzeichnet, daß ein Polyvinylalkohol mit einem Reaktionspartner umgesetzt wird, der eine Gruppe $R_4$ enthält, und mit mindestens einem Reaktionspartner, der eine Gruppe $-RN^{\oplus}R_1R_2R_3A^{\ominus}$ oder $-RNR_1R_2$ enthält.

2. Verfahren nach Anspruch 1, dadurch gekennzeichnet, daß die mit Sauerstoff verbundenen

angehängten Gruppen mindestens teilweise aus einem Gemisch von Gruppen —$RN^{\oplus}R_1R_2R_3A^{\ominus}$ und von Gruppen —$RNR_1R_2$ und $R_4$ bestehen, wobei die Gruppen R, $R_1$, $R_2$, $R_3$, $R_4$ und A wie im Anspruch 1 definiert sind.

3. Verfahren nach einem der vorhergehenden Ansprüche, dadurch gekennzeichnet, daß die Polyvinylalkohol-Hauptkette ein zahlenmäßig durchschnittliches Molekulargewicht von mindestens 2000 hat.

4. Verfahren nach einem der vorhergehenden Ansprüche, dadurch gekennzeichnet, daß das Gesamtformelgewicht aller Gruppen $R_4$ 0,5 bis 50 Gewichtsprozent des Polymers beträgt.

5. Verfahren nach einem der vorhergehenden Ansprüche, dadurch gekennzeichnet, daß das Verhältnis des Gesamtformelgewichts aller Gruppen —$RNR_1R_2$ zu dem Gesamtformelgewicht aller Gruppen $RN^+R_1R_2R_3A^-$ im Bereich von 0 bis 90 Gew.% liegt.

6. Verfahren nach einem der vorhergehenden Ansprüche, dadurch gekennzeichnet, daß R ein Radikal ist, das aus der Gruppe ausgewählt ist, die aus

$$—CH_2—CHOH—CH_2 \text{ un } —[CH_2CH(CH_2N^+R_1R_2R_3A^-)O]_eCH_2CH(OH)CH_2—$$

besteht, wobei $R_1$, $R_2$ und $R_3$ Alkyl- oder Aralkylradikale mit 1 bis 20 Kohlenstoffatomen sind und $A^-$ ein Halogenidion oder ein (niederes Alkyl)—$SO_4^-$ und e=1 bis 20 ist.

7. Verfahren nach einem der vorhergehenden Ansprüche, dadurch gekennzeichnet, daß die Gruppe $RN^{\oplus}R_1R_2R_3A^{\ominus}$ die Gruppe —$COCH_2N^+R_1R_2R_3A^-$ ist.

8. Verfahren nach einem der vorhergehenden Ansprüche, dadurch gekennzeichnet, daß die angehängten Gruppen R—$NR_1R_2$ aus der Gruppe ausgewählt sind, die aus

$$—CH_2CHOH—CH_2NR_1R_2 \text{ und } [—CH_2CH(CH_2NR_1R_2)—O]_eCH_2—CH(OH)—CH_2NR_1R_2$$

besteht, wobei $R_1$ und $R_2$ Alkyl- oder Aralkylradikale mit 1 bis 20 Kohlenstoffatomen sind und e=1 bis 20 ist.

9. Verfahren nach einem der vorhergehenden Ansprüche, dadurch gekennzeichnet, daß die angehängten Gruppen —R—$NR_1R_2$ Gruppen —$COCH_2NR_1R_2$ sind, in denen $R_1$ und $R_2$ Alkyl- oder Arylalkylradikale mit 1 bis 20 Kohlenstoffatomen sind.

10. Verfahren nach einem der vorhergehenden Ansprüche, dadurch gekennzeichnet, daß $R_1$, $R_2$ und $R_3$ aus der Gruppe ausgewählt sind, die aus

—$CH_3$, —$CH_2CH_3$, —$CH_2CH_2CH_3$, 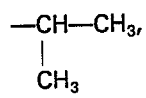 und —$CH_2Ph$

besteht, wobei Ph Phenyl ist.

11. Verfahren nach einem der vorhergehenden Ansprüche, dadurch gekennzeichnet, daß —$R_4$ aus der Gruppe ausgewählt ist, die aus 1,9-Dihydroxyoctadecan und 1,10-Dihydroxyoctadecan, Octadecansäure, 9-Hydroxyoctadecansäure, 10-Hydroxyoctadecansäure, 9,10-Dihydroxyoctadecansäure und den Salzen und Estern dieser Säuren besteht.

12. Verfahren nach Anspruch 11, dadurch gekennzeichnet, daß eine oder beide der Hydroxygruppen des 1,9- oder 1,10-Dihydroxyoctadecans durch Sauerstoff mit Gruppen der allgemeinen Formel —R—$NR_1R_2$ und/oder —R—$N^+R_1R_2R_3A^-$ verbunden sind, in denen R, $R_1$, $R_2$, $R_3$ und $A^-$ dieselbe Bedeutung haben wie im Anspruch 1.

13. Verfahren nach Anspruch 1, dadurch gekennzeichnet, daß $R_4$ aus der Gruppe ausgewählt ist, die aus

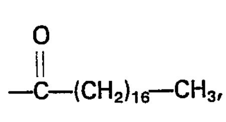, 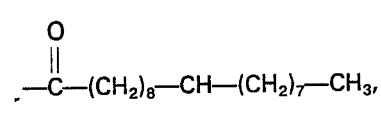, 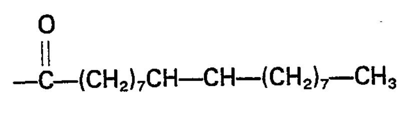

und

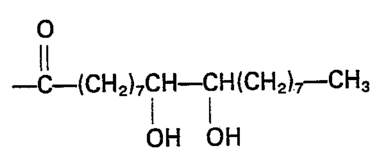

besteht.

14. Verfahren nach Anspruch 11, dadurch gekennzeichnet, daß ein Teil oder alle der Hydroxygruppen von $R_4$ durch Sauerstoff mit Gruppen —R—$NR_1R_2$ und/oder —R—$N^+R_1R_2R_3A^-$ verbunden sind, wobei die Gruppen R, $R_1$, $R_2$, $R_3$ und $A^-$ dieselben sind wie im Anspruch 1.

15. Verfahren nach einem der vorhergehenden Ansprüche, dadurch gekennzeichnet, daß die angehängten Gruppen mindestens teilweise aus einem Gemisch aus mindestens zwei Gruppen $RN^{\oplus}R_1R_2R_3A^{\ominus}$, $RNR_1R_2$ und $R_4$ bestehen, in denen R, $R_1$, $R_2$, $R_3$, $R_4$ und $A^{\ominus}$ die im Anspruch 1 angegebenen

Bedeutungen haben, $R_1$, $R_2$ und $R_3$ Methylgruppen sind und der Stickstoffgehalt im Bereich von 0,05 bis 3 Gew.% beträgt.

16. Verfahren nach Anspruch 1, dadurch gekennzeichnet, daß die angehängten Gruppen mindestens teilweise aus Gruppen $RN^+R_1R_2R_3A^\ominus$ bestehen, in denen $R_1$, $R_2$ und $R_3$ Methylgruppen sind und der Stickstoffgehalt im Bereich von 0,01 bis 3 Gew.% liegt.

17. Verfahren nach Anspruch 1, bei dem folgende Schritte durchgeführt wurden: (a) äquimolare Mengen Epichlorhydrin und wäßriges Trimethylamin werden bei Temperaturen unter 25°C umgesetzt; (b) es wird eine wäßrige Lösung von Polyvinylalkohol mit einem zahlenmäßig durchschnittlichen Molekulargewicht von mindestens 2000 hergestellt, die eine Base in einer katalytisch wirksamen Menge enthält, (c) die in den Schritten (a) und (b) hergestellten Lösungen werden miteinander vermischt, und danach wird ein Alkyl- oder substituiertes Alkylepoxid mit dem Produkt des Schritts (c) vermischt.

18. Verfahren nach Anspruch 1 mit folgenden Schritten: (a) ein Alkyl- oder substituiertes Alkylepoxid wird bei einer Temperatur von 30 bis 90°C mit einer wäßrigen Lösung eines Polyvinylalkohols umgesetzt, der ein zahlenmäßig durchschnittliches Molekulargewicht im Bereich von 25 000 bis 150 000 hat, wobei die Lösung Kaliumhydroxid in einer katalytisch wirksamen Menge enthält; (b) Epichlorhydrin und eine wäßrige Lösung von Trimethylamin werden bei einer Temperatur unter 25°C umgesetzt, (c) die in den Schritten (a) und (b) erhaltenen Produkte werden bei einer Temperatur von 40 bis 90°C umgesetzt.

19. Verfahren nach Anspruch 1 mit folgenden Schritten: (a) (Chlorhydroxypropyl)trialkylammoniumchlorid in wäßriger oder kristalliner Form wird bei einer Temperatur von 40 bis 90°C mit einer wäßrigen Lösung eines Polyvinylalkohols mit einem zahlenmäßig durchschnittlichen Molekulargewicht von mindestens 2000 in Gegenwart einer katalytisch wirksamen Menge Kaliumhydroxid umgesetzt; und (b) das im Schritt (a) erhaltene Reaktionsprodukt wird mit einem Alkylepoxid umgesetzt.

20. Verfahren nach Anspruch 1 mit folgenden Schritten: (a) ein Alkyl- oder substituiertes Alkylepoxid wird bei 30 bis 90°C in Gegenwart einer katalytisch wirksamen Menge Kaliumhydroxid mit einem wäßrigen Polyvinylalkohol umgesetzt, der ein zahlenmäßig durchschnittliches Molekulargewicht von 2000 bis 200 000 hat; (b) das im Schritt (a) erhaltene Reaktionsprodukt wird bei einer Temperatur von 40 bis 90°C mit (Chlorhydroxypropyl)trimethylammoniumchlorid in Form einer wäßrigen Lösung oder kristalliner Form umgesetzt.

21. Verfahren nach Anspruch 1 mit folgenden Schritten: (a) in wäßriger Lösung wird ein Polyvinylalkohol mit einem zahlenmäßig durchschnittlichen Molekulargewicht im Bereich von 25 000 bis 150 000 bei einer Temperatur von 40 bis 90°C mit einer wäßrigen Lösung von 2,3-Epoxypropyltrialkylammoniumhalogenid in Gegenwart einer katalytisch wirksamen Menge einer alkalischen Substanz umgesetzt, wobei das Verhältnis der Molanzahl des Ammoniumhalogenids zu der Molanzahl der Hydroxylgruppe des Polyvinylalkoholpolymers im Bereich von 0,003 bis 1 liegt; (b) das im Schritt (a) erhaltene Produkt wird mit einem Alkylepoxid umgesetzt.

22. Verfahren nach Anspruch 1 mit folgenden Schritten: (a) ein Alkyl- oder substituiertes Alkylepoxid wird bei 30 bis 90°C in Gegenwart einer katalytisch wirksamen Menge Kaliumhydroxid mit einem wäßrigen Polyvinylalkohol mit einem zahlenmäßig durchschnittlichen Molekulargewicht im Bereich von 25 000 bis 150 000 umgesetzt, bis die Reaktion vollständig durchgeführt worden ist; (b) eine wäßrige Lösung von 2,3-Epoxypropyltrialkylammoniumhalogenid wird bei einer Temperatur von 40 bis 90°C mit dem im Schritt (a) erhaltenen Produkt in Gegenwart einer katalytisch wirksamen Menge einer Base umgesetzt, und zwar in solchen Mengen, daß das Verhältnis der Molanzahl des Ammoniumhalogenids zu der Molanzahl der Hydroxylgruppen des Polyvinylalkoholpolymers im Bereich von 0,003 bis 1 liegt.

23. Verfahren nach Anspruch 1 mit folgenden Schritten: (a) ein Polyvinylalkohol mit einem zahlenmäßig durchschnittlichen Molekulargewicht von 25 000 bis 150 000 wird bei einer Temperatur von 150°C mit einem Trimethylethoxycarbonylmethylammoniumhalogenid in einer dipolaren aprotischen Lösung in Gegenwart einer katalytischen wirksamen Menge Magnesiumacetat umgesetzt; (b) das im Schritt (a) erhaltene Produkt wird mit einem Alkyl- oder einem substituierten Alkylepoxid umgesetzt.

24. Verfahren nach Anspruch 1 mit folgenden Schritten: (a) ein Polyvinylalkohol mit einem zahlenmäßig durchschnittlichen Molekulargewicht von 25 000 bis 150 000 wird mit einem Alkylepoxid in einer wäßrigen Lösung umgesetzt, eine eine katalytisch wirksame Menge Alkalihydroxid enthält; (b) das im Schritt (a) erhaltene Reaktionsprodukt wird bei einer Temperatur von 70 bis 150°C in Gegenwart einer katalytisch wirksamen Menge Magnesiumacetat mit Trimethylethoxycarbonylmethylammoniumhalogenid in einer dipolaren aprotischen Lösung umgesetzt.

25. Verfahren nach Anspruch 19 oder 22, dadurch gekennzeichnet, daß das Ammoniumhalogenid (Chlorhydroxypropyl)-trimethylammoniumchlorid und das Alkylepoxid 9,10-Epoxy-octadecan-1-ol ist.

26. Verfahren nach Anspruch 19 oder 22, dadurch gekennzeichnet, daß das Ammoniumhalogenid 2,3-Epoxypropyltrimethylammoniumchlorid und das Alkylepoxid 9,10-Epoxyoctadecan-1-ol ist.

27. Verfahren nach Anspruch 1, dadurch gekennzeichnet, daß das nach einem der Ansprüche 17, 18 und 19 erhaltene Produkt bei einer Temperatur von 30 bis 90°C mit einem Alkylepoxid umgesetzt worden ist.

28. Verfahren nach Anspruch 1, dadurch gekennzeichnet, daß das nach Anspruch 25 erhaltene Produkt in einem wäßrigen Reaktionsmedium mit einem Alkyl- oder substituierten Alkylepoxid umgesetzt wird.

29. Verfahren nach Anspruch 1, in dem eine wäßrige Lösung eines Polyvinylalkoholpolymers mit einem zahlenmäßig durchschnittlichen Molekulargewicht von 25 000 bis 150 000 bei einer Temperatur von

40 bis 90°C in Gegenwart einer katalytisch wirksamen Menge Säure mit 9,10-Epoxyoctadecanol und 2,3-Epoxypropyltrialkylammoniumhalogenid umgesetzt wird, wobei das Verhältnis der Gesamtanzahl der Mole des Epoxid zu der Anzahl der Mole der Hydroxylgruppen des Polyvinylalkoholpolymers im Bereich von 0,003 bis 1 liegt.

30. Verfahren nach Anspruch 1, in dem die gewünschte Zusammensetzung aus einem wäßrigen Reaktionsgemisch durch Zusatz eines Nichtlösungsmittels ausgefällt wird, das aus der Gruppe ausgewählt ist, die aus Aceton und Methanol besteht.

31. Verfahren nach einem der vorhergehenden Ansprüche, die in wäßriger Lösung durch Dialyse oder Ultrafiltration gereinigt wird.

32. Wäßrige Dispersion, die 0,1 bis 30 Gew.% einer Zusammensetzung nach einem der vorhergehenden Ansprüche enthält.

33. Wäßrige Zusammensetzung zum Konditionieren der Haut, die 0,1 bis 5 Gew.% einer Zusammensetzung enthält, die ein modifiziertes Polyvinylalkoholpolymer enthält, das eine Polyvinylalkohol-Hauptkette besitzt, and die mit Sauerstoff verbundene Gruppen angehängt sind, die ausgewählt sind aus

a) $—RN^{\oplus}R_1R_2R_3A^{\ominus}$ und

b) einer Kombination von $RNR_1R_2$ und $R_4$,

wobei R aus der Gruppe ausgewählt ist, die aus einem Alkylen-, einem substituierten Alkylen- oder einem Acylenradikal besteht, $R_1$, $R_2$, $R_3$ Alkyl- oder Arylalkylradikale mit 1 bis 20 Kohlenstoffatomen sind, $R_4$ aus der Gruppe ausgewählt ist, die aus den Alkyl-, Alkylaryl-, substituierten Alkyl-, Alkylaryl- oder Arylalkylradikalen besteht, die Hydroxy- oder Carboxylgruppen oder eine Kombination von Hydroxy- und Carboxylgruppen tragen, und $A^-$ ein Anion ist, ferner 2 bis 5 Gew.% eines Emulgators und 2 bis 5 Gew.% eines Erweichungsmittels.

34. Wäßrige Zusammensetzung zum Konditionieren der Haare, die 0,1 bis 5 Gew.% einer Zusammensetzung enthält, die ein modifiziertes Polyvinylalkoholpolymer enthält, das eine Polyvinylalkohol-Hauptkette besitzt, an die mit Sauerstoff verbundene Gruppen angehängt sind, die ausgewählt sind aus

a) $—RN^{\oplus}R_1R_2R_3A^{\ominus}$ und

b) einer Kombination von $RNR_1R_2$ und $R_4$,

wobei R aus der Gruppe ausgewählt ist, die aus einem Alkylen-, einem substituierten Alkylen- oder einem Acylenradikal besteht, $R_1$, $R_2$, $R_3$ Alkyl- oder Arylalkylradikale mit 1 bis 20 Kohlenstoffatomen sind, $R_4$ aus der Gruppe ausgewählt ist, die aus den Alkyl-, Alkylaryl-, substituierten Alkyl- Alkylaryl- oder Arylalkylradikalen besteht, die Hydroxy- oder Carboxylgruppen oder eine Kombination von Hydroxy- und Carboxylgruppen tragen, und $A^-$ ein Anion ist.

35. Kosmetische Zusammensetzung, die 0,5 bis 5 Gew.% einer Zusammensetzung enthält, die ein modifiziertes Polyvinylalkoholpolymer enthält, das eine Polyvinylalkohol-Hauptkette besitzt, an die mit Sauerstoff verbundene Gruppen angehängt sind, die ausgewählt sind aus

a) $—RN^{\oplus}R_1R_2R_3A^{\ominus}$ und

b) einer Kombination von Gruppen $RNR_1R_2$ und $R_4$,

wobei R aus der Gruppe ausgewählt ist, die aus einem Alkylen-, einem substituierten Alkylen- oder einem Acylenradikal besteht, $R_1$, $R_2$, $R_3$ Alkyl- oder Arylalkylradikale mit 1 bis 20 kohlenstoffatomen sind, $R_4$ aus der Gruppe ausgewählt ist, die aus den Alkyl-, Alkylaryl-, substituierten Alkyl-, Alkylaryl- oder Arylalkylradikalen besteht, die Hydroxy- oder Carboxylgruppen oder eine Kombination von Hydroxy- und Carboxylgruppen tragen, und $A^-$ ein Anion ist.

36. Zusammensetzung nach einem der Ansprüche 32 bis 35, dadurch gekennzeichnet, daß das Polyvinylalkoholpolymer ein Copolymer ist, das bis zu 25 Gew.% Polyvinylacetat, Polyvinylacrylat, Polyvinylmethacrylat und Polyvinylpyrrolidon enthält.

**Revendications pour les Etats Contractants: BE, CH, DE, FR, GB, IT, LI, LU, NL, SE**

1. Polymère d'alcool polyvinylique modifié comprenant une chaîne de base alcool polyvinylique ayant en tant que ligands pendants au moins un groupe $R_4$ lié par un atome d'oxygène et au moins un autre groupe pendant lié par un atome d'oxygène et choisi parmi

a) $—RN^+R_1R_2R_3A^-$

b) $—RNR_1R_2$,

où

R est choisi parmi l'ensemble constitué par les radicaux alkylène, alkylène substitué et acylène,

$R_1$, $R_2$ et $R_3$ représentent des radicaux alkyle ou aralkyle ayant de 1 à 20 atomes de carbone,

$R_4$ est choisi parmi l'ensemble constitué par les groupes alkyle, les groupes alkylaryle, les groupes alkyle substitués, les groupes alkylaryle substitués et les groupes arylalkyle, comportant des groupes hydroxyle ou carboxyle ou une combinaison de groupes hydroxyle et carboxyle,

$A^-$ est un anion,

ledit polymère ayant une teneur total en azote comprise dans la gamme allant de 0,01 à 7,0% en poids.

2. Polymère d'alcool polyvinylique modifié par au moins un atome d'azote quaternaire suivant la revendication 1, dans lequel ledit groupe pendant lié par un atome d'oxygène comprend un mélange de groupes $—RN^+R_1R_2R_3A^-$ et de groupes $—RNR_1R_2$ et $R_4$ où R, $R_1$, $R_2$, $R_3$, $R_4$ et $A^-$ sont définis comme indiqué dans la revendication 1.

3. Composition de polymère suivant l'une quelconque des revendications précédentes, dans laquelle ladite chaîne de base alcool polyvinylique a un poids moléculaire moyen en nombre d'au moins 2 000.

4. Composition de polymère suivant l'une quelconque des revendications précédentes, dans laquelle les poids cumulés de tous les groupes $R_4$ de la formule représentent 0,5—50% en poids dudit polymère.

5. Composition suivant l'une quelconque des revendications précédentes, dans laquelle le rapport des poids totaux de tous les groupes $—RNR_1R_2$ de la formule aux poids totaux desdits groupes $—RN^+R_1R_2R_3A^-$ de la formule est compris dans la gamme allant de 0 à 90% en poids.

6. Composition suivant l'une quelconque des revendications précédentes, dans laquelle R est un groupe choisi parmi l'ensemble constitué par

$$—CH_2—CHOH—CH_2— \quad \text{et} \quad —[CH_2CH(CH_2N^+R_1R_2R_3A^-)O]_eCH_2CH(OH)CH_2—$$

où $R_1$, $R_2$ et $R_3$ sont des radicaux alkyle ou aralkyle en $C_1—C_{20}$, et $A^-$ est un ion halogénure ou (alkyle inférieur) $SO_4^-$, et e a pour valeur 1 à 20.

7. Composition suivant l'une quelconque des revendications précédentes, dans laquelle ledit groupe $—RN^+R_1R_2R_3A^-$ est le groupe $—COCH_2N^+R_1R_2R_3A^-$.

8. Composition suivant l'une quelconque des revendications précédentes, dans laquelle les groupes pendants $—RNR_1R_2$ sont choisis parmi l'ensemble constitué par les groupes

$$—CH_2CH(OH)CH_2NR_1R_2 \quad \text{et} \quad —[CH_2CH(CH_2NR_1R_2)—O]_eCH_2CH(OH)CH_2NR_1R_2$$

où $R_1$ et $R_2$ sont des radicaux alkyle ou aralkyle en $C_1—C_{20}$ et e a pour valeur 1 à 20.

9. Composition suivant l'une quelconque des revendications précédentes, dans laquelle les groupes pendants $—RNR_1R_2$ sont des groupes $—COCH_2NR_1R_2$ où $R_1$ et $R_2$ sont des radicaux alkyle ou aralkyle en $C_1—C_{20}$.

10. Composition suivant l'une quelconque des revendications précédentes, dans laquelle $R_1$, $R_2$ et $R_3$ sont choisis parmi l'ensemble constitué par $—CH_3$, $—CH_2CH_3$, $—CH_2CH_2CH_3$, $—CH(CH_3)_2$ ou $—CH_2Ph$ où Ph est phényle.

11. Composition suivant l'une quelconque des revendications précédentes, dans laquelle $R_4$ est choisi parmi l'ensemble constitué par le 1,9-dihydroxyoctadécane, le 1,10-dihydroxyoctadécane, l'acide octadécanoïque, l'acide 9-hydroxyoctadécanoïque, l'acide 10-hydroxyoctadécanoïque, l'acide 9,10-dihydroxyoctadécanoïque, les sels et esters desdits acides.

12. Composition suivant la revendication 11, dans laquelle l'un ou les deux groupes OH des 1,9- et 1,10-dihydroxyoctadécanes sont chacun reliés par une liaison oxy aux groupes de formule générale $—RNR_1R_2$ et/ou $—RN^+R_1R_2R_3A^-$, où R, $R_1$, $R_2$, $R_3$ et $A^-$ sont définis comme indiqué dans la revendication 1.

13. Composition suivant la revendication 1, dans laquelle $R_4$ est choisi parmi l'ensemble constitué par

$$—C(=O)—(CH_2)_{16}—CH_3, \quad —C(=O)—(CH_2)_8—CH—(CH_2)_7—CH_3, \quad —C(=O)—(CH_2)_7CH=CH(CH_2)_7—CH_3$$

et

$$—C(=O)—(CH_2)_7—CH(OH)CH(OH)—(CH_2)_7—CH_3.$$

14. Composition suivant la revendication 11, dans laquelle une partie ou la totalité des groupes OH présents dans $R_4$ est reliée par une liaison oxy aux groupes $—RNR_1R_2$ et/ou $—RN^+R_1R_2R_3A^-$ où les groupes R, $R_1$, $R_2$, $R_3$ et $A^-$ sont définis comme indiqué dans la revendication 1.

15. Composition suivant l'une quelconque des revendications précédentes, dans laquelle lesdits groupes pendants comprennent un mélange d'au moins deux des groupes $—RN^+R_1R_2R_3A^-$ et des groupes $—RNR_1R_2$ et $R_4$ où R, $R_1$, $R_2$, $R_3$, $R_4$ et $A^-$ sont définis comme indiqué dans la revendication 1, les groupes $R_1$, $R_2$ et $R_3$ étant notamment $CH_3$ et la teneur en azote étant comprise dans la gamme allant de 0,05 à 3% en poids.

16. Composition suivant la revendication 1, dans laquelle les groupes pendants comprennent des groupes $—RN^+R_1R_2R_3A^-$ où $R_1$, $R_2$ et $R_3$ sont $CH_3$ et la teneur en azote est comprise dans la gamme allant de 0,01 à 3% en poids.

17. Composition de polymère suivant la revendication 1, caractérisée en ce qu'elle est préparée suivant les étapes comprenant: (a) la réaction, suivant des quantités équimolaires, de l'épichlorhydrine avec la triméthylamine à une température inférieure à 25°C, (b) la formation d'une solution aqueuse d'alcool polyvinylique ayant un poids moléculaire moyen en nombre d'au moins 2 000 contenant une quantité catalytique d'une base, (c) le mélange des solutions des étapes (a) et (b) puis le mélange d'un alkyl-époxyde, ou d'un alkyl-époxide où le groupe alkyle est substitué, avec le produit de l'étape (c).

18. Composition de polymère suivant la revendication 1, caractérisée en ce qu'elle est préparé suivant les étapes comprenant: (a) la réaction d'un alkyl-époxyde, ou alkyl-époxyde où le groupe alkyle est substitué, avec une solution aqueuse d'un alcool polyvinylique ayant un poids moléculaire moyen en nombre de 25 000—150 000 contenant une quantité catalytique de potasse à une température de 30—90°C, (b) la réaction de l'épichlorhydrine avec une solution aqueuse de triméthylamine à une température inférieure à 25°C, (c) la réaction du produit de l'étape (a) avec le produit de l'étape (b) à une température de 40—90°C.

19. Composition de polymère suivant la revendication 1, caractérisée en ce qu'elle est préparée suivant les étapes comprenant: (a) l'addition de chlorure de (chlorohydroxypropyl)trialkylammonium sous forme aqueuse ou cristalline à une solution aqueuse d'un alcool polyvinylique ayant un poids moléculaire moyen en nombre d'au moins 2 000, en présence d'une quantité catalytique de potasse à une température de 40—90°C, et (b) la réaction du produit de réaction obtenu à l'étape (a) avec un alkyl-époxyde.

20. Composition de polymère suivant la revendication 1, caractérisée en ce qu'elle est préparée suivant les étapes comprenant: (a) la réaction d'un alkyl-époxyde, ou d'un alkyl-époxyde où le groupe alkyle est substitué, avec un alcool polyvinylique aqueux ayant un poids moléculaire moyen en nombre de 2 000—200 000, en présence d'une quantité catalytique de potasse à 30—90°C, et (b) la réaction du produit de ladite étape (a) avec le chlorure de (chlorohydroxypropyl)triméthylammonium en solution aqueuse ou sous forme crystalline, à une température de 40—90°C.

21. Composition de polymère suivant la revendication 1, caractérisée en ce qu'elle est préparée suivant les étapes comprenant: (a) la réaction en solution aqueuse d'un alcool polyvinylique ayant un poids moléculaire moyen en nombre de 25 000—150 000 avec une solution aqueuse d'halogénure de 2,3-époxypropyltrialkylammonium en présence d'une quantité catalytique de potasse, à une température de 40—90°C, de telle façon que le rapport molaire halogénure d'ammonium/groupe OH sur ledit polymère d'alcool polyvinylique soit compris dans la gamme allant de 0,003 à 1, et (b) la réaction dudit produit de l'étape (a) avec un alkyl-époxyde.

22. Composition de polymère suivant la revendication 1, caractérisée en ce qu'elle est préparée suivant les étapes comprenant: (a) la réaction d'un alkyl-époxyde, ou d'un alkyl-époxyde où le groupe alkyle est substitué, avec un alcool polyvinylique aqueux ayant un poids moléculaire moyen en nombre de 25 000—150 000, en présence d'une quantité catalytique de potasse, à une température de 30—90°C, jusqu'à ce que ladite région soit complète, et (b) la réaction d'une solution aqueuse d'halogénure de 2,3-époxypropyltrialkylammonium avec le produit de l'étape (a) à une température de 40—90°C, en présence d'une quantité catalytique d'une base, le rapport molaire halogénure d'ammonium/groupe OH sur ledit polymère dl'alcool polyvinylique étant compris dans la gamme allant de 0,003 à 1.

23. Composition de polymère suivant la revendication 1, caractérisée en ce qu'elle est préparée suivant les étapes comprenant: (a) la réaction d'un alcool polyvinylique ayant un poids moléculaire moyen en nombre de 25 000—150 000 avec l'halogénure de triméthyléthoxycarbonylméthylammonium en solution aprotique dipolaire, en présence d'une quantité catalytique d'acétate de magnésium, à une température de 150°C, et (b) la réaction du produit de l'étape (a) avec un alkyl-époxyde ou d'un alkyl-époxyde où le groupe alkyle est substitué.

24. Composition de polymère suivant la revendication 1, caractérisée en ce qu'elle est préparée suivant les étapes comprenant: (a) la réaction d'un alcool polyvinylique ayant un poids moléculaire moyen en nombre de 25 000—150 000 avec un alkyl-époxyde en solution aqueuse, en présence d'une quantité catalytique d'hydroxyde alcalin, et (b) la réaction du produit de réaction de l'étape (a) avec l'halogénure de triméthyléthoxycarbonylméthylammonium en solution aprotique dipolaire, en présence d'une quantité catalytique d'acétate de magnésium, à une température de 70—150°C.

25. Composition suivant la revendication 19 ou 22, caractérisée en ce que dans son procédé de préparation ledit halogénure d'ammonium est le chlorure de (chlorohydroxypropyl)triméthylammonium et ledit alkyl-époxyde est le 9,10-époxyoctadécane-1-ol.

26. Composition suivant la revendication 19 ou 22, caractérisée en ce que dans son procédé de préparation ledit halogénure d'ammonium est le chlorure de 2,3-époxypropyltriméthylammonium et ledit alkyl-époxyde est le 9,10-époxyoctadécane-1-ol.

27. Composition de polymère suivant la revendication 1, dans laquelle le produit de l'une quelconque des revendications 17, 18 et 19 a été mis en réaction avec un alkyl-époxyde à une température de 30—90°C.

28. Composition de polymère suivant la revendication 1, dans laquelle le produit de la revendication 22 a été mis en réaction avec un alkyl-époxyde, ou d'un alkyl-époxyde où le groupe alkyle est substitué, dans un milieu de réaction aqueux.

29. Composition de polymère suivant la revendication 1, caractérisée en ce qu'elle est préparée par réaction d'un polymère d'alcool polyvinylique ayant un poids moléculaire moyen en nombre de 25 000—150 000 avec le 9,10-époxyoctadécanol et un halogénure de 2,3-époxypropyltrialkylammonium, à une température de 40—90°C, en présence d'une quantité catalytique d'acide, le rapport molaire époxyde total/total de groupes OH sur le polymère d'alcool polyvinylique étant compris dans la gamme allant de 0,003 à 1.

30. Composition de polymère suivant la revendication 1, caractérisée en ce qu'elle est séparée à partir d'un mélange réactionnel aqueux par précipitation au moyen d'un composant non-solvant choisi parmi l'ensemble constitué par l'acétone et le méthanol.

31. Composition suivant l'une quelconque des revendications précédentes, caractérisée en ce qu'elle est purifiée en solution aqueuse par dialyse ou ultrafiltration.

32. Dispersion aqueuse contenant 0,1—30% en poids d'une composition suivant l'une quelconque des revendications précédentes.

33. Composition aqueuse pour le conditionnement de la peau comprenant 0,1—5% en poids de polymère d'alcool polyvinylique modifié ayant des groupes pendants liés par une liaison oxy et choisis parmi

a) $-RN^+R_1R_2R_3A^-$, et

b) une combinaison de groupes $RNR_1R_2$ et $R_4$,

où R, $R_1$, $R_2$, $R_3$, $R_4$ et $A^-$ sont définis comme indiqué dans la revendication 1, 2—5% en poids d'un agent émulsifiant et 2—5% en poids d'un agent émollient.

34. Composition aqueuse pour le conditionnement des cheveux comprenant 0,1—5% en poids de polymère d'alcool polyvinylique modifié ayant des groupes pendants liés par une liaison oxy et choisis parmi

a) $-RN^+R_1R_2R_3A^-$, et

b) une combinaison de groupes $RNR_1R_2$ et $R_4$,

où R, $R_1$, $R_2$, $R_3$, $R_4$ et $A^-$ sont définis comme indiqué dans la revendication 1.

35. Composition cosmétique contenant 0,5—5% en poids de polymère d'alcool polyvinylique modifié ayant des groupes pendants liés par une liaison oxy et choisis parmi

a) $-RN^+R_1R_2R_3A^-$, et

b) une combinaison de groupes $RNR_1R_2$ et $R_4$,

où R, $R_1$, $R_2$, $R_3$, $R_4$ et $A^-$ sont définis comme indiqué dans la revendication 1.

36. Composition pharmaceutique contenant 0,5—5% en poids de polymère d'alcool polyvinylique modifié ayant des groupes pendants liés par une liaison oxy et choisis parmi

a) $-RN^+R_1R_2R_3A^-$, et

b) une combinaison de groupes $RNR_1R_2$ et $R_4$,

où R, $R_1$, $R_2$, $R_3$, $R_4$ et $A^-$ sont définis comme indiqué dans la revendication 1.

37. Polymère d'alcool polyvinylique amino-modifié, utile dans le domaine du conditionnement de la peau et les formulations cosmétiques et pharmaceutiques, comprenant une chaîne de base polyvinylique ayant une pluralité de groupes pendants liés par une liaison oxy et répondant à la formule générale:

$$-RNR_1R_2$$

dans laquelle

R est choisi l'ensemble constitué par les radicaux alkylène, alkylène substitué et acylène,

$R_1$ et $R_2$ sont choisis parmi l'ensemble constitué par les radicaux alkyle et aralkyle en $C_1$—$C_{20}$,

ledit polymère ayant une teneur total en azote située dans la gamme allant de 0,01 à 7% en poids.

38. Composition suivant l'une quelconque des revendications précédentes, dans laquelle l'alcool polyvinylique est un copolymère ayant au plus 25 parties en poids de poly(acétate de vinyle), polyvinylacrylate, polyvinylméthacrylate et polyvinylpyrrolidone.

**Revendications pour l'Etat Contractant: AT**

1. Procédé pour la préparation d'un alcool polyvinylique modifié comprenant une chaîne de base alcool polyvinylique ayant en tant que ligands pendants au moins un groupe $R_4$ lié par un atome d'oxygène et au moins un autre groupe pendant lié par un atome d'oxygène et choisi parmi

a) $-RN^+R_1R_2R_3A^-$

b) $-RNR_1R_2$,

où

R est choisis parmi l'ensemble constitué par les radicaux alkylène, alkylène substitué et acylène,

$R_1$, $R_2$ et $R_3$ représentent des radicaux alkyle ou aralkyle ayant de 1 à 20 atomes de carbone,

$R_4$ est choisi parmi l'ensemble constitué par les groupes alkyle, les groupes alkylaryle, les groupes alkyle substitués, les groupes alkylaryle substitués ou les groupes arylalkyle, comportant des groupes hydroxyle ou carboxyle ou une combinaison de groupes hydroxyle et carboxyle,

$A^-$ est un anion,

ledit polymère ayant une teneur totale en azote comprise dans la gamme allant de 0,01 à 7,0% en poids, ledit procédé étant caractérisé en ce que l'on fait réagir un alcool polyvinylique avec un réactif contenant au moins un groupe $R_4$ et au moins un réactif contenant un groupe $-RN^+R_1R_2R_3A^-$ ou $-RNR_1R_2$.

2. Procédé suivant la revendication 1, dans lequel ledit groupe pendant lié par un atome d'oxygène comprend en outre un mélange de groupes $-RN^+R_1R_2R_3A^-$ et de groupes $-RNR_1R_2$ et $R_4$, où R, $R_1$, $R_2$, $R_3$, $R_4$ et $A^-$ sont définis comme indiqué dans la revendication 1.

3. Procédé suivant l'une quelconque des revendications précédentes, dans lequel ladite chaîne de base alcool polyvinylique a un poids moléculaire moyen en nombre d'au moins 2 000.

4. Procédé suivant l'une quelconque des revendications précédentes, dans lequel les poids cumulés de tous les groupes $R_4$ de la formule représentent 0,5—50% en poids dudit polymère.

5. Procédé suivant l'une quelconque des revendications précédentes, dans lequel le rapport des poids totaux de tous les groupes $-RNR_1R_2$ de la formule aux poids totaux desdits groupes $-RN^+R_1R_2R_3A^-$ de la formule est compris dans la gamme allant de 0 à 90% en poids.

6. Procédé suivant l'une quelconque des revendications précédentes, dans lequel R est un groupe choisi parmi l'ensemble constitué par

$$-CH_2-CHOH-CH_2- \quad \text{et} \quad -[CH_2CH(CH_2N^+R_1R_2R_3A^-)O]_eCH_2CH(OH)CH_2-,$$

où $R_1$, $R_2$ et $R_3$ sont des radicaux alkyle ou aralkyle en $C_1$—$C_{20}$, et $A^-$ est un ion halogénure ou (alkyle (inférieur) $SO_4^-$, et e a pour valeur 1 à 20.

7. Procédé suivant l'une quelconque des revendications précédentes, dans lequel ledit groupe $—RN^+R_1R_2R_3A^-$ est le groupe $—COCH_2N^+R_1R_2R_3A^-$.

8. Procédé suivant l'une quelconque des revendications précédentes, dans lequel les groupes pendants $—RNR_1R_2$ sont choisis parmi l'ensemble constitué par les groupes

$$—CH_2CH(OH)CH_2NR_1R_2 \quad \text{et} \quad —[CH_2CH(CH_2NR_1R_2)—O]_eCH_2CH(OH)CH_2NR_1R_2$$

où $R_1$ et $R_2$ sont des radicaux alkyle ou aralkyle en $C_1$—$C_{20}$ et e a pour valeur 1 à 20.

9. Procédé suivant l'une quelconque des revendications précédentes, dans lequel les groupes pendants $—RNR_1R_2$ sont des groupes $—COCH_2NR_1R_2$ où $R_1$ et $R_2$ sont des radicaux alkyle ou aralkyle en $C_1$—$C_{20}$.

10. Procédé suivant l'une quelconque des revendications précédentes, dans lequel $R_1$, $R_2$ et $R_3$ sont choisis parmi l'ensemble constitué par $—CH_3$, $—CH_2CH_3$, $—CH_2CH_2CH_3$, $—CH(CH_3)_2$ ou $—CH_2Ph$ où Ph est phényle.

11. Procédé suivant l'une quelconque des revendications précédentes, dans lequel $R_4$ est choisi parmi l'ensemble constitué par le 1,9-dihyroxyoctadécane, le 1,10-dihydroxyoctadécane, l'acide octadécanoïque, l'acide 9-hydroxyoctadécanoïque, l'acide 10-hydroxyoctadécanoïque, l'acide 9,10-dihydroxyoctadécanoïque, les sels et esters desdits acides.

12. Procédé suivant la revendication 11, dans lequel l'un ou les deux groupes OH des 1,9- et 1,10-dihydroxyoctadécanes sont reliés par une liaison oxy aux groupes de formule générale $—RNR_1R_2$ et/ou $—RN^+R_1R_2R_3A^-$, où R, $R_1$, $R_2$, $R_3$ et $A^-$ sont définis comme indiqué dans la revendication 1.

13. Procédé suivant la revendication 1, dans lequel $R_4$ est choisi parmi l'ensemble constitué par

$$—C(=O)—(CH_2)_{16}—CH_3, \quad —C(=O)—(CH_2)_8—CH—(CH_2)_7—CH_3 \quad —C(=O)—(CH_2)_7CH{=}CH(CH_2)_7—CH_3$$

et

$$—C(=O)—(CH_2)_7—CH(OH)CH(OH)—(CH_2)_7—CH_3.$$

14. Procédé suivant la revendication 11, dans lequel une partie ou la totalité des groupes OH présents dans $R_4$ est reliée par une liaison oxy aux groupes $—RNR_1R_2$ et/ou $—RN^+R_1R_2R_3A^-$, où les groupes R, $R_1$, $R_2$, $R_3$ et $A^-$ sont définis comme indiqué dans la revendication 1.

15. Procédé suivant l'une quelconque des revendications précédentes, dans lequel lesdits groupes pendants comprennent un mélange d'au moins deux des groupes $—RN^+R_1R_2R_3A^-$, $—RNR_1R_2$ et $R_4$ où R, $R_1$, $R_2$, $R_3$, $R_4$ et $A^-$ sont définis comme indiqué dans la revendication 1, les groupes $R_1$, $R_2$ et $R_3$ étant notamment $CH_3$ et la teneur en azote étant comprise dans la gamme allant de 0,05 à 3% en poids.

16. Procédé suivant la revendication 1, dans lequel les groupes pendants comprennent des groupes $—RN^+R_1R_2R_3A^-$ où $R_1$, $R_2$ et $R_3$ sont $CH_3$ et la teneur en azote est comprise dans la gamme allant de 0,01 à 3% en poids.

17. Procédé suivant la revendication 1, comprenant les étapes suivantes: (a) la réaction, suivant des quantités équimolaires, de l'épichlorhydrine avec la triméthylamine à une température inférieure à 25°C, (b) la formation d'une solution aqueuse d'alcool polyvinylique ayant un poids moléculaire moyen en nombre d'au moins 2 000 contenant une quantité catalytique d'une base, (c) le mélange des solutions des étapes (a) et (b) puis le mélange d'un alkyl-époxyde, ou d'un alkyl-époxyde où le groupe alkyle est substitué, avec le produit de l'étape (c).

18. Procédé suivant la revendication 1, comprenant les étapes suivantes: (a) la réaction d'un alkyl-époxyde, ou alkyl-époxyde où le groupe alkyle est substitué, avec une solution aqueuse d'un alcool polyvinylique ayant un poids moléculaire moyen en nombre de 25 000—150 000 contenant une quantité catalytique de potasse à une température de 30—90°C, (b) la réaction de l'épichlorhydrine avec une solution aqueuse de triméthylamine à une température inférieure à 25°C, (c) la réaction du produit de l'étape (a) avec le produit de l'étape (b) à une température de 40—90°C.

19. Procédé suivant la revendication 1, comprenant les étapes suivantes: (a) l'addition de chlorure de (chlorohydroxypropyl)trialkylammonium sous forme aqueuse ou cristalline à une solution aqueuse d'un alcool polyvinylique ayant un poids moléculaire moyen en nombre d'au moins 2 000, en présence d'une quantité catalytique de potasse à une température de 40—90°C, et (b) la réaction du produit de réaction obtenu à l'étape (a) avec un alkyl-époxyde.

20. Procédé suivant la revendication 1, comprenant les étapes suivantes: (a) la réaction d'un alkyl-époxyde, ou d'un alkyl-époxyde où le groupe alkyle est substitué, avec un alcool polyvinylique aqueux ayant un poids moléculaire moyen en nombre de 2 000—200 000, en présence d'une quantité catalytique de potasse à 30—90°C, et (b) la réaction du produit de ladite étape (a) avec le chlorure de (chlorohydroxypropyl)triméthylammonium en solution aqueuse ou sous forme cristalline, à une température de 40—90°C.

21. Procédé suivant la revendication 1, comprenant les étapes suivantes: (a) la réaction en solution aqueuse d'un alcool polyvinylique ayant un poids moléculaire moyen en nombre de 25 000—150 000 avec une solution aqueuse d'halogénure de 2,3-époxypropyltrialkylammonium en présence d'une quantité

catalytique de potasse, à une température de 40—90°C, de telle façon que le rapport molaire halogénure d'ammonium/groupe OH sur ledit polymère d'alcool polyvinylique soit compris dans la gamme allant de 0,003 à 1, et (b) la réaction dudit produit de l'étape (a) avec un alkyl-époxyde.

22. Procédé suivant la revendication 1, comprenant les étapes suivantes: (a) la réaction d'un alkyl-époxyde, ou d'un alkyl-époxyde où le groupe alkyle est substitué, avec un alcool polyvinylique aqueux ayant un poids moléculaire moyen en nombre de 25 000—150 000, en présence d'une quantité catalytique de potasse, à une température de 30—90°C, jusqu'à ce que ladite réaction soit complète, et (b) la réaction d'une solution aqueuse d'halogénure de 2,3-époxypropyltrialkylammonium avec le produit de l'étape (a) à une température de 40—90°C, en présence d'une quantité catalytique d'une base, le rapport molaire halogénure d'ammonium/groupe OH sur ledit polymère d'alcool polyvinylique étant compris dans la gamme allant de 0,003 à 1.

23. Procédé suivant la revendication 1, comprenant les étapes suivantes: (a) la réaction d'un alcool polyvinylique ayant un poids moléculaire moyen en nombre de 25 000—150 000 avec l'halogénure de triméthyléthoxycarbonylméthylammonium en solution aprotique dipolaire, en présence d'une quantité catalytique d'acétate de magnésium, à une température de 150°C, et (b) la réaction du produit de l'étape (a) avec un alkyl-époxyde ou d'un alkyl-époxyde où le groupe alkyle est substitué.

24. Procédé suivant la revendication 1, comprenant les étapes suivantes: (a) la réaction d'un alcool polyvinylique ayant un poids moléculaire moyen en nombre de 25 000—150 000 avec un alkyl-époxyde en solution aqueuse, en présence d'une quantité catalytique d'hydroxyde alcalin, et (b) la réaction du produit de réaction de l'étape (a) avec l'halogénure de triméthyléthoxycarbonylméthylammonium en solution aprotique dipolaire, en présence d'une quantité catalytique d'acétate de magnésium, à une température de 70—150°C.

25. Procédé suivant la revendication 19 ou 22, dans lequel ledit halogénure d'ammonium est le chlorure de (chlorohydroxypropyl)triméthylammonium et ledit alkyl-époxyde est le 9,10-époxyoctadécane-1-ol.

26. Procédé suivant la revendication 19 ou 22, dans lequel ledit halogénure d'ammonium est le chlorure de 2,3-époxypropyltriméthylammonium et ledit alkyl-époxyde est le 9,10-époxyoctadécane-1-ol.

27. Procédé suivant la revendication 1, dans lequel le produit de l'une quelconque des revendications 17, 18 et 19 a été mis en réaction avec un alkyl-époxyde à une température de 30—90°C.

28. Procédé suivant la revendication 1, dans lequel le produit de la revendication 22 a été mis en réaction avec un alkyl-époxyde ou d'un alkyl-époxyde, où le groupe alkyle est substitué, dans un milieu de réaction aqueux.

29. Procédé suivant la revendication 1, comprenant la réaction d'un polymère d'alcool polyvinylique ayant un poids moléculaire moyen en nombre de 25 000—150 000 avec le 9,10-époxyoctadécanol et un halogénure de 2,3-époxypropyltrialkylammonium, à une température de 40—90°C, en présence d'une quantité catalytique d'acide, le rapport molaire époxyde total/total groupes OH sur le polymère d'alcool polyvinylique étant compris dans la gamme allant de 0,003 à 1.

30. Procédé suivant la revendication 1, dans lequel la composition désirée est séparée à partir d'un mélange réactionnel aqueux par précipitation au moyen d'un composant non-solvant choisi parmi l'ensemble constitué par l'acétone et le méthanol.

31. Procédé suivant l'une quelconque des revendications précédentes dans lequel la composition est purifiée en solution aqueuse par dialyse ou ultrafiltration.

32. Dispersion aqueuse contenant 0,1—30% en poids d'une composition obtenue suivant le procédé de l'une quelconque des revendications précédentes.

33. Composition aqueuse pour le conditionnement de la peau comprenant 0,1—5% en poids de polymère d'alcool polyvinylique modifié ayant des groupes pendants liés par une liaison oxy et choisis parmi

a) $—RN^+R_1R_2R_3A^-$, et

b) une combinaison de groupes $RNR_1R_2$ et $R_4$,

où R est choisi parmi l'ensemble constitué par les radicaux alkylène, les radicaux alkylène substitué et les radicaux acylène, $R_1$, $R_2$ et $R_3$ sont des radicaux alkyle ou aralkyle en $C_1$—$C_{20}$, $R_4$ est choisi parmi l'ensemble constitué par les radicaux alkyle, les radicaux alkylaryle, les radicaux alkyle, alkylaryle ou arylalkyle substitués comportant des groupes hydroxyle ou carboxyle ou une combinaison de groupes hydroxyle et carboxyle, $A^-$ est un anion; 2—5% en poids d'un agent émulsifiant et 2—5% en poids d'une agent émollient.

34. Composition aqueuse pour le conditionnement des cheveux comprenant 0,1—5% en poids de polymère d'alcool polyvinylique modifié ayant des groupes pendants liés par une liaison oxy et choisis parmi

a) $—RN^+R_1R_2R_3A^-$, et

b) une combinaison de $RNR_1R_2$ et $R_4$,

où R est choisi parmi l'ensemble constitué par les radicaux alkylène, les radicaux alkylène substitué et les radicaux acylène, $R_1$, $R_2$ et $R_3$ sont des radicaux alkyle ou aralkyle en $C_1$—$C_{20}$, $R_4$ est choisi parmi l'ensemble constitué par les radicaux alkyle, les radicaux alkylaryle, les radicaux alkyle ou alkylaryle substitués et les radicaux arylalkyle, comportant des groupes hydroxyle ou carboxyle ou une combinaison de groupes hydroxyle et carboxyle, $A^-$ est un anion.

35. Composition cosmétique contenant 0,5—5% en poids de polymère d'alcool polyvinylique modifié ayant des groupes pendants liés par une liaison oxy et choisis parmi

a) $—RN^+R_1R_2R_3A^-$, et

b) une combinaison de groupes $RNR_1R_2$ et $R_4$,

où R est choisi parmi l'ensemble constitué par les radicaux alkylène, alkylène substitué et les radicaux acylène, $R_1$, $R_2$ et $R_3$ sont des radicaux alkyle ou aralkyle en $C_1—C_{20}$, $R_4$ est choisi parmi l'ensemble constitué par les radicaux alkyle, les radicaux alkylaryle, les radicaux alkyle ou alkylaryle substitués et les radicaux arylalkyle, comportant des groupes hydroxyle ou carboxyle ou une combinaison de groupes hydroxyle et carboxyle, $A^-$ est un anion.

36. Composition suivant l'une quelconque des revendications 32 à 35, dans laquelle l'alcool polyvinylique est un copolymère ayant au plus 25 parties en poids de poly(acétate de vinyle), polyvinylacrylate, polyvinylméthacrylate et polyvinylpyrrolidone.